# EUROPÄISCHE PATENTANMELDUNG

(11) **EP 4 124 259 A1**
(43) Veröffentlichungstag der Anmeldung: **01.02.2023**
(21) Anmeldenummer: 21188046.3
(22) Anmeldetag: 27.07.2021
(51) Int. Cl.: A46B 13/00, A46B 11/06, A61B 17/00

(54) **CHIRURGISCHE REINIGUNGSVORRICHTUNG**

(71) Anmelder: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(57) **Zusammenfassung**

Die Erfindung betrifft eine chirurgische Reinigungsvorrichtung aufweisend
ein Gehäuse (1),
eine Antriebswelle (2), die drehbar gelagert ist, die ein hinteres Ende (3) und ein vorderes Ende (4) aufweist,
einen Bürstenkopf (5), an dem eine Vielzahl elastisch verformbarer Borsten (6) befestigt sind, wobei der Bürstenkopf (5) außerhalb des Gehäuses (1) angeordnet ist,
eine Pumpe (18) zum Pumpen einer Spülflüssigkeit, wobei die Pumpe (18) mit der Antriebswelle (2) verbunden ist und anzutreiben ist,
ein Rohr (7), wobei der Bürstenkopf (5) an einem vorderen Ende (8) des Rohrs (7) angeordnet ist, wobei das Rohr (7) linear beweglich gelagert ist und wobei der Bürstenkopf (5) im Bereich der elastisch verformbaren Borsten (6) zumindest eine Ausspritzöffnung aufweist, wobei das Rohr (7) flüssigkeitsdurchlässig durch die zumindest eine Ausspritzöffnung in Richtung der elastisch verformbaren Borsten (6) mündet,
ein Getriebe (10), das mit dem vorderen Ende (4) der Antriebswelle (2) und mit einem hinteren Ende (12) des Rohrs (7) verbunden ist und das die Drehbewegung der Antriebswelle (2) in eine oszillierende lineare Bewegung des Rohrs (7) und des Bürstenkopfs (5) übersetzt.

Die Erfindung betrifft auch ein Verfahren zum Betreiben einer solchen chirurgischen Reinigungsvorrichtung.

## Beschreibung

Die Erfindung betrifft eine chirurgische Reinigungsvorrichtung und ein Verfahren zum Betreiben einer solchen chirurgischen Reinigungsvorrichtung.

Gegenstand der Erfindung ist dabei insbesondere eine einmalig zu verwendende chirurgische Reinigungsvorrichtung, die sowohl zur Reinigung von infizierten Gelenkendoprothesen als auch von infizierten Knochen- und Weichgewebearealen bestimmt und geeignet ist. Mit der chirurgischen Reinigungsvorrichtung können aber auch andere gebrauchte Werkzeuge und Bestecke, die im Rahmen einer medizinischen Operation (OP) verwendet wurden, gereinigt werden.

In der orthopädischen Chirurgie müssen leider in einem gewissen Umfang septische Revisionen von mit Mikroorganismen infizierten Gelenkendoprothesen vorgenommen werden. Dabei werden die infizierten Gelenkendoprothesen explantiert und das infizierte beziehungsweise nekrotische Gewebe abgetragen. Dieses Abtragen von infiziertem/nekrotischem Gewebe wird als Debridement bezeichnet. Ebenso müssen die explantierten Gelenkendoprothesen gereinigt werden.

In der Medizin sind gegenwärtig mit Druckluft und mit elektrischer Energie betriebene Antriebseinheiten bekannt. Derartige externe Antriebseinheiten sind in Operationssälen (OP-Sälen) vorhanden und können für eine Vielzahl von Zwecken eingesetzt und genutzt werden.

In der septischen Knochenchirurgie ist die Reinigung von debridierten Knochen- und Weichgewebearealen bei der Revision von infizierten Gelenkendoprothesen und die Reinigung von infizierten Osteosyntheseplatten durch Lavagierung Stand der Technik. Dafür werden medizinische Spülvorrichtungen, sogenannte Lavage-Systeme, eingesetzt. Als Flüssigkeiten sind physiologische Kochsalzlösung, Ringer-Lösung und Ringer-Lactat-Lösung üblich. Das Ziel der Lavagierung besteht in der Entfernung von Resten des infizierten Gewebes, von Biofilmresten, von Blut und von Wundsekret. Lavage-Systeme sind seit langem bekannt. Exemplarisch sind dafür Vorrichtungen und Antriebe hierfür gemäß den Druckschriften DE 10 2011 018 708 A1, EP 2 939 699 B1, EP 3 187 208 B1, EP 3 574 852 A1, EP 3 742 316 A1, US 4 583 531 A, US 5 542 918 A, US 5 779 702 A, US 6 059 754 A, US 2003/0083681 A1, US 2005/0084395 A1 und US 2019/0151531 A1 genannt.

Das Debridieren und Reinigen kann durch Wundspülung mit sogenannten Lavage-Systemen sowie durch Ausschneiden, Raspeln, Sägen und auch Bürsten erfolgen. Eine Vorrichtung zum Bürsten und Sägen ist beispielsweise aus der EP 3 741 316 A1, der EP 3 574 852 A1 und der nicht vorveröffentlichten EP 21154092 bekannt. Die verwendeten Vorrichtungen sind nach dem Debridement mit Geweberesten und mit mikrobiellen Keimen kontaminiert. Für eine neue Verwendung müssen diese Instrumente sorgfältig gereinigt und anschließend sterilisiert werden. Dabei muss sich das medizinische Personal vor einer Kontamination beziehungsweise einer Infektion durch Übertragung von mikrobiellen Keimen während der Reinigungsarbeiten schützen. Daher ist es wünschenswert, eine kostengünstige chirurgische Reinigungsvorrichtung bereitzustellen, die nach einmaliger Verwendung ohne aufwendige und nicht gefahrlose Reinigungsschritte mit dem üblichen OP-Abfall zusammen entsorgt werden könnte. Es wäre dann besonders aus Gründen des Ressourcen- und Umweltschutzes aber auch aus Kostengründen sinnvoll, wenn für den Antrieb keine Batterien, Akkumulatoren und Elektromotoren notwendig wären, die Buntmetalle enthalten und daher gegebenenfalls aufwendig recycelt werden müssten. Nach einer mechanischen Reinigung oder Bearbeitung muss häufig zusätzlich ein Debridement erfolgen, um entstandene und möglicherweise infektiöse Rückstände wie Späne, die bei der mechanischen Bearbeitung entstanden sind, zu entfernen. Hierfür wird ein weiterer Arbeitsschritt benötigt und alle verwendeten Vorrichtungen müssen anschließend aufwendig desinfiziert beziehungsweise entsorgt werden.

Die Aufgabe der vorliegenden Erfindung besteht also darin, die Nachteile des Stands der Technik zu überwinden. Insbesondere besteht die Aufgabe der Erfindung in der Entwicklung einer einmalig zu verwendenden chirurgischen Reinigungsvorrichtung, die zur Reinigung von infizierten Gelenkendoprothesen und von infizierten Knochen- und Weichgewebearealen bestimmt ist. Diese Vorrichtung soll möglichst einfach und kostengünstig zu fertigen sein. Weiterhin soll die Vorrichtung durch Ethylenoxid oder Gammasterilisation zu sterilisieren sein. Die chirurgische Reinigungsvorrichtung soll einfach in der Anwendung sein und möglichst wenig notwendige Arbeitsschritte erfordern.

Die chirurgische Reinigungsvorrichtung soll mit einer OP-üblichen, mit Batterien oder Akkumulatoren betriebenen Antriebseinheit temporär verbunden werden können, wobei die verbundene Antriebseinheit die chirurgische Reinigungsvorrichtung antreibt. Die Reinigung von Gewebearealen soll mechanisch durch Einwirkung von vibrierenden elastisch verformbaren Borsten und gleichzeitig durch ein oder mehrere nicht gepulste oder gepulste Spülflüssigkeitsstrahlen erfolgen, wobei die Spülflüssigkeit von außen durch eine Schlauchzuleitung in die Vorrichtung geleitet werden soll. Die Spülflüssigkeit soll dann durch eine in der Vorrichtung enthaltene Pumpe angesaugt und anschließend in Form von Sprühstrahlen wieder ausgestoßen werden. Diese Spülflüssigkeitsstrahlen sollen aus der Reinigungsvorrichtung im Bereich der Bürsten austreten. Die Reinigungsvorrichtung muss so beschaffen sein, dass diese nach beendeter Reinigung wie üblicher Klinikabfall entsorgt werden kann.

Es wäre vorteilhaft, wenn die Reinigungsvorrichtung nach der Verwendung hygienisch durch Verbrennung entsorgt werden kann. Die chirurgische Reinigungsvorrichtung muss, um chirurgisch anwendbar zu sein, steril sein. Die chirurgische Reinigungsvorrichtung soll möglichst nach beendeter Reinigung zusammen mit anderen Klinikabfällen durch Verbrennung entsorgt werden können. Die chirurgische Reinigungsvorrichtung soll durch eine für medizinische Operationen übliche Antriebseinheit angetrieben werden können.

Die der vorliegenden Erfindung zugrundeliegenden Aufgaben werden gelöst durch eine chirurgische Reinigungsvorrichtung aufweisend
ein Gehäuse,
eine Antriebswelle, wobei die Antriebswelle drehbar in dem Gehäuse gelagert ist, wobei die Antriebswelle ein hinteres Ende zur Verbindung mit einer Antriebseinheit aufweist und wobei die Antriebswelle ein vorderes Ende aufweist, das dem hinteren Ende gegenüberliegend angeordnet ist,
einen Bürstenkopf, wobei der Bürstenkopf eine Vielzahl elastisch verformbarer Borsten aufweist, die an dem Bürstenkopf befestigt sind, und wobei der Bürstenkopf außerhalb des Gehäuses angeordnet ist,
eine Pumpe zum Pumpen einer Spülflüssigkeit, wobei die Pumpe mit der Antriebswelle verbunden ist und von der Antriebswelle anzutreiben ist, ein Rohr, wobei der Bürstenkopf an einem vorderen Ende des Rohrs angeordnet ist, wobei das Rohr linear beweglich gegen das Gehäuse gelagert ist und wobei der Bürstenkopf im Bereich der elastisch verformbaren Borsten zumindest eine Ausspritzöffnung aufweist, wobei das Rohr flüssigkeitsdurchlässig durch die zumindest eine Ausspritzöffnung in Richtung der elastisch verformbaren Borsten mündet, und
ein Getriebe, wobei das Getriebe mit dem vorderen Ende der Antriebswelle und mit einem hinteren Ende des Rohrs verbunden ist und wobei das Getriebe die Drehbewegung der Antriebswelle in eine oszillierende lineare Bewegung des Rohrs und des Bürstenkopfs übersetzt.

Das hintere Ende des Rohrs ist dem vorderen Ende des Rohrs gegenüberliegend angeordnet.

Das Rohr kann bevorzugt mit einem Spülflüssigkeitsreservoir verbunden sein.

Bevorzugt ragt das Rohr mit dem vorderen Ende aus dem Gehäuse heraus, während das hintere Ende des Rohrs innerhalb des Gehäuses angeordnet ist.

Damit das Rohr flüssigkeitsdurchlässig durch die zumindest eine Ausspritzöffnung des Bürstenkopfs in Richtung der elastisch verformbaren Borsten münden kann, müssen im Bürstenkopf flüssigkeitsdurchlässige Leitungen vorgesehen sein, die mit dem vorderen Ende des Rohrs einerseits und mit der zumindest einen Ausspritzöffnung andererseits flüssigkeitsdurchlässig verbunden sind. Bevorzugt wird dies dadurch realisiert, dass der Bürstenkopf ein Hohlkörper mit einem inneren Hohlraum ist, wobei das Rohr flüssigkeitsdurchlässig mit dem Hohlraum des Bürstenkopfs verbunden ist und wobei die zumindest eine Ausspritzöffnung durch zumindest eine Bohrung in einer Wandung des Bürstenkopfs realisiert ist, die den Hohlraum mit der Umgebung des Bürstenkopfs verbindet. Dabei kann es auch vorgesehen sein, dass zumindest eine Borste der elastisch verformbaren Borsten innen hohl ausgeführt ist und mit der Bohrung flüssigkeitsdurchlässig verbunden ist.

Die erfindungsgemäße chirurgische Reinigungsvorrichtung zeichnet sich dadurch aus, dass sie keinen Motor und keinen elektrischen Energiespeicher aufweist. Dadurch kann die chirurgische Reinigungsvorrichtung kostengünstig als hygienisches Wegwerfprodukt für medizinische Anwendungen gefertigt werden und insbesondere aus leicht und rückstandsfrei verbrennbarem Kunststoff bestehen.

Es kann also vorgesehen sein, dass die chirurgische Reinigungsvorrichtung aus Kunststoffen gefertigt ist, insbesondere aus solchen Kunststoffen, die durch Verbrennung entsorgt werden können.

Um chirurgisch anwendbar zu sein, ist es wesentlich, dass die chirurgische Reinigungsvorrichtung steril ist oder zumindest alle Oberflächen der Reinigungsvorrichtung sterilisierbar sind, wie beispielsweise durch Gamma-Strahlen-Sterilisation oder durch Sterilisation mit Ethylenoxid. Hierzu kann bevorzugt vorgesehen sein, dass die chirurgischen Reinigungsvorrichtung in einer für Ethylenoxid durchlässigen Verpackung enthalten ist.

Das hintere Ende der Antriebswelle kann als Anschluss zum Verbinden mit einer externen Antriebseinheit ausgebildet sein, insbesondere als eine Vierkantstange, Sechskantstange oder allgemein als Kantstange ausgebildet sein.

Bevorzugt kann vorgesehen sein, dass die Pumpe im Inneren des Gehäuses angeordnet ist.

Es kann vorgesehen sein, dass die chirurgische Reinigungsvorrichtung eine erste Flüssigkeitsleitung aufweist, wobei die erste Flüssigkeitsleitung mit dem hinteren Ende des Rohrs flüssigkeitsdurchlässig verbunden ist und am hinteren Ende des Rohrs fixiert ist und an einem dem hinteren Ende des Rohrs gegenüberliegenden Ende relativ zum Gehäuse fixiert ist, wobei bevorzugt die erste Flüssigkeitsleitung ein flexibler beweglicher Schlauch ist, der an einem ersten Ende des Schlauchs fest mit dem hinteren Ende des Rohrs verbunden ist.

Hierdurch kann im Betrieb der chirurgischen Reinigungsvorrichtung eine Spülflüssigkeit durch die erste Flüssigkeitsleitung in das oszillierende Rohr eingespeist werden, wobei die erste Flüssigkeitsleitung die lineare oszillierende Bewegung aufnimmt und einen gegen das Gehäuse fixierten Zugang für die Spülflüssigkeit bereitstellt.

Bevorzugt kann hierzu das Rohr über die erste Flüssigkeitsleitung mit einem Spülflüssigkeitsreservoir verbunden oder verbindbar sein.

Des Weiteren kann vorgesehen sein, dass die chirurgische Reinigungsvorrichtung eine zweite Flüssigkeitsleitung aufweist, wobei die zweite Flüssigkeitsleitung derart mit der Pumpe verbunden ist, dass eine Spülflüssigkeit von der Pumpe durch die zweite Flüssigkeitsleitung in die Pumpe ansaugbar ist und wobei die erste Flüssigkeitsleitung derart mit der Pumpe verbunden ist, dass eine Spülflüssigkeit von der Pumpe aus der Pumpe heraus in die zweite Flüssigkeitsleitung drückbar ist.

Hierdurch kann die sich drehende Antriebswelle nicht nur die lineare oszillierende Bewegung des Bürstenkopfs antreiben, sondern auch den Spülflüssigkeitsstrahl antreiben. Die chirurgische Reinigungsvorrichtung benötigt dann neben der Antriebseinheit zum Antreiben der Drehung der Antriebswelle nur noch ein Spülflüssigkeitsreservoir, aus dem die Pumpe die Spülflüssigkeit mit der zweiten Flüssigkeitsleitung absaugen kann.

Bei chirurgischen Reinigungsvorrichtungen mit einer zweiten Flüssigkeitsleitung kann vorgesehen sein, dass die Pumpe derart mit der Antriebswelle verbunden ist, dass zumindest bei einem Stillstand der Antriebswelle die zweite Flüssigkeitsleitung in der Pumpe von der erste Flüssigkeitsleitung flüssigkeitsundurchlässig getrennt ist oder abgeriegelt ist, wobei bevorzugt bei einer Drehbewegung der Antriebswelle die zweite Flüssigkeitsleitung flüssigkeitsleitend mit der erste Flüssigkeitsleitung verbunden ist.

Die Trennung oder Abriegelung kann durch mechanisches Blockieren innerhalb der Pumpe erfolgen. Hiermit kann verhindert werden, dass kontaminierte Spülflüssigkeit durch die erste Flüssigkeitsleitung und die Pumpe in die zweite Flüssigkeitsleitung vordringen kann und dort anschließend ein Reservoir für die Spülflüssigkeit kontaminiert, ohne dass hierfür ein separates oder zusätzliches Ventilsystem notwendig wäre. Dadurch kann die chirurgische Reinigungsvorrichtung als kostengünstiges und hygienisches Wegwerfprodukt aufgebaut werden. Die Pumpe ist mit der Antriebswelle verbunden, die bei Nichtdrehen der Antriebswelle den Spülflüssigkeitsstrom der Spülflüssigkeit ausgehend von der zweiten Flüssigkeitsleitung durch mechanisches Blockieren der Verbindung zwischen der zweiten Flüssigkeitsleitung und der ersten Flüssigkeitsleitung abriegelt und bei Drehbewegung der Antriebswelle den Spülflüssigkeitsstrom von der zweiten Flüssigkeitsleitung zur ersten Flüssigkeitsleitung freigibt. Dadurch ist es möglich, durch den Verzicht von Ventilen und Rückschlagventilen die Vorrichtung einfach und kostengünstig zu halten.

Bevorzugt kann das Rohr über die zweite Flüssigkeitsleitung, die Pumpe und die erste Flüssigkeitsleitung mit einem Spülflüssigkeitsreservoir verbunden oder verbindbar sein.

Ferner kann vorgesehen sein, dass die Pumpe eine selbstansaugende Pumpe ist, die unmittelbar mit der Antriebswelle verbunden ist, wobei die selbstansaugende Pumpe in dem Gehäuse angeordnet ist.

Hierdurch wird ein besonders einfacher Aufbau erreicht. Zudem wird so sichergestellt, dass gleichzeitig mit der oszillierenden Bewegung des Bürstenkopfs ein Ausstoß von Spülflüssigkeit stattfindet, um so die beiden Reinigungswirkungen zu kombinieren. Eine selbstansaugende Pumpe in der chirurgischen Reinigungsvorrichtung hat den Vorteil, dass die chirurgische Reinigungsvorrichtung Spülflüssigkeiten, wie physiologische Kochsalzlösung, Ringer-Lösung oder Ringer-Lactat-Lösung, selbst ansaugen kann und diese dann in Form von Spülflüssigkeitsstrahlen aus der chirurgischen Reinigungsvorrichtung ausgestoßen wird.

Auch kann vorgesehen sein, dass die Pumpe eine Impellerpumpe ist, die auf der Antriebswelle angeordnet ist, wobei die Impellerpumpe einen Rotor mit Impellerflügeln aufweist, wobei der Rotor mit den Impellerflügeln kraft- und/oder formschlüssig mit der Antriebswelle verbunden ist.

Hierdurch wird ein besonders einfacher und kostengünstiger Aufbau erreicht. Gleichzeitig sind die zweite Flüssigkeitsleitung und die erste Flüssigkeitsleitung durch die Impellerflügel in der Impellerpumpe voneinander getrennt und so wird eine Kontamination der Spülflüssigkeit durch verunreinigte gebrauchte Spülflüssigkeit auf einfache Weise und ohne Ventilsystem verhindert.

Die Pumpe ist mit der Antriebswelle verbunden, die bei Nichtdrehen der Antriebswelle den Spülflüssigkeitsstrom ausgehend von der zweiten Flüssigkeitsleitung durch mechanisches Blockieren der Verbindung zwischen der zweiten Flüssigkeitsleitung und dem Rohr abriegelt und bei Drehbewegung der Antriebswelle den Spülflüssigkeitsstrom von der zweiten Flüssigkeitsleitung zum Rohr freigibt.

Bevorzugt kann auch vorgesehen sein, dass das Getriebe im Inneren des Gehäuses angeordnet ist.

Hierdurch ist die Bewegung des Getriebes durch Störungen von außerhalb der chirurgischen Reinigungsvorrichtung geschützt.

Des Weiteren kann vorgesehen sein, dass das Gehäuse ein Griffstück zum Halten der chirurgischen Reinigungsvorrichtung mit einer Hand aufweist und wobei das Gehäuse eine Öffnung für das hintere Ende der Antriebswelle aufweist.

Hierdurch ist die chirurgische Reinigungsvorrichtung gefahrlos und bequem manuell einsetzbar. Das Gehäuse kann neben dem Schutz der darin befindlichen Teile, wie der Antriebswelle, dem Getriebe und der ersten Flüssigkeitsleitung zusätzlich dazu genutzt werden, einen geschützten Hohlraum für andere Teile der chirurgischen Reinigungsvorrichtung zu bilden.

Gemäß einer bevorzugten Weiterbildung kann vorgesehen sein, dass die Antriebswelle durch eine separate Antriebseinheit drehbar und anzutreiben ist, wobei die separate Antriebseinheit hierzu mit dem hinteren Ende der Antriebswelle verbunden oder verbindbar ist, insbesondere lösbar verbunden oder verbindbar ist.

Die separate Antriebseinheit kann eine externe Antriebseinheit sein.

Hierdurch benötigt die chirurgische Reinigungsvorrichtung keinen eigenen Motor und keinen Energiespeicher. Dadurch kann die chirurgische Reinigungsvorrichtung zum einmaligen Gebrauch gefertigt werden und als hygienisches Wegwerfprodukt durch Verbrennen entsorgt werden.

Zum Entfernen der gebrauchten Spülflüssigkeit kann vorgesehen sein, dass die chirurgische Reinigungsvorrichtung eine Absaugeinrichtung mit zumindest einer Absaugöffnung im Bereich der elastisch verformbaren Borsten aufweist, wobei die Absaugeinrichtung eine Absaugleitung aufweist, die an eine Unterdruckquelle anschließbar ist oder angeschlossen ist.

Hierdurch kann die gebrauchte Spülflüssigkeit abgesaugt und von dem zu reinigenden Objekt entfernt werden.

Dabei kann bevorzugt vorgesehen sein, dass die Absaugeinrichtung an einen Abscheider zum Abscheiden von festen Bestandteilen aus der gebrauchten Spülflüssigkeit angeschlossen oder anschließbar ist und/oder die Unterdruckquelle eine Vakuumpumpe aufweist, die ein Teil der chirurgischen Reinigungsvorrichtung ist und die von der Antriebswelle antreibbar ist.

Hierdurch muss die gebrauchte Spülflüssigkeit nicht auf andere Weise mit einer separaten Vorrichtung entfernt beziehungsweise aufbereitet werden. Bevorzugt kann vorgesehen sein, dass ein Absaugrohr der Absaugeinrichtung das Rohr bereichsweise umschließt oder parallel zum Rohr geführt ist, wobei besonders bevorzugt das Absaugrohr an dem Rohr befestigt ist.

Es kann auch vorgesehen sein, dass das Rohr zumindest teilweise von einem äußeren Rohr umgeben ist, wobei der Zwischenraum zwischen der Innenwand des äußeren Rohrs und der Außenwand des Rohrs mit einer externen Absauganlage verbunden ist.

Des Weiteren kann vorgesehen sein, dass die chirurgische Reinigungsvorrichtung aus Kunststoff gefertigt ist, wobei bevorzugt die gesamte chirurgische Reinigungsvorrichtung durch Verbrennung rückstandsfrei entsorgbar ist.

Hierdurch kann die chirurgische Reinigungsvorrichtung als hygienisches Wegwerfprodukt im medizinischen Bereich verwendet werden und zudem kostengünstig gefertigt werden.

Ferner kann vorgesehen sein, dass die chirurgische Reinigungsvorrichtung eine flexible Antriebswellenverlängerung aufweist, wobei die flexible Antriebswellenverlängerung an das hintere Ende der Antriebswelle angeschlossen oder anschließbar ist und wobei die flexible Antriebswellenverlängerung an eine externe Antriebseinheit anschließbar ist, so dass die Antriebswelle mit der externen Antriebseinheit über die flexible Antriebswellenverlängerung gegen das Gehäuse drehbar ist.

Hierdurch kann die externe Antriebseinheit von den elastisch verformbaren Borsten der chirurgischen Reinigungsvorrichtung und damit von dem Ort der Reinigung entfernt platziert werden, so dass die externe Antriebseinheit, die einen Motor enthält, nicht verschmutzt und daher nicht aufwendig gereinigt werden muss.

Des Weiteren kann vorgesehen sein, dass der Bürstenkopf ein Hohlkörper ist und die zumindest eine Ausspritzöffnung einen Innenraum des Hohlkörpers mit der Umgebung im Bereich der elastisch verformbaren Borsten verbindet, wobei das Rohr über den Innenraum des Hohlkörpers in die zumindest eine Ausspritzöffnung mündet.

Hierdurch kann die Spülflüssigkeit durch das Rohr und den hohlen Bürstenkopf zu der zumindest einen Ausspritzöffnung geleitet werden. Gleichzeitig ist der Aufbau einfach und es kann Gewicht und Material gespart werden, das nach erfolgter Reinigung nicht verbrannt werden muss.

Es kann auch vorgesehen sein, dass die chirurgische Reinigungsvorrichtung ein Rückstellelement aufweist, mit dem das Rohr in Richtung der Antriebswelle gedrückt wird, wobei bevorzugt das Rückstellelement zumindest eine Spiralfeder ist, wobei besonders bevorzugt die zumindest eine Spiralfeder um das Rohr herum und/oder um einen Schaft zur Verbindung des Rohrs mit dem Getriebe herum angeordnet ist und gegen das Gehäuse gelagert ist.

Hierdurch kann sichergestellt werden, dass das Rohr immer in Kontakt mit der Antriebswelle steht und so das Getriebe stets seine Funktion erfüllt. Das Rückstellelement bewirkt dabei eine lineare Rückwärtsbewegung nach einer durch das Getriebe und der Antriebswelle erzwungenen Vorwärtsbewegung.

Die Spiralfeder kann aus Edelstahl, aus Kunststoffen oder auch aus nichtkorrodierenden Metalllegierungen beliebiger Zusammensetzung gefertigt werden.

Gemäß einer bevorzugten Weiterbildung der vorliegenden Erfindung kann auch vorgesehen sein, dass das Getriebe eine Scheibe an dem vorderen Ende der Antriebswelle oder an dem hinteren Ende des Rohrs aufweist, wobei die Scheibe eine gegen die Drehachse der Antriebswelle geneigte Oberfläche aufweist, oder
das Getriebe eine erste Scheibe an dem vorderen Ende der Antriebswelle und eine zweite Scheibe an dem hinteren Ende des Rohrs aufweist, wobei die erste Scheibe und die zweite Scheibe jeweils eine gegen die Drehachse der Antriebswelle geneigte Oberfläche aufweisen, wobei die geneigten Oberflächen der ersten Scheibe und der zweiten Scheibe zueinander ausgerichtet sind.

Auf diese Weise kann ein besonders einfaches und kostengünstiges Getriebe realisiert werden. Der Aufbau mit der Scheibe oder der ersten Scheibe und der zweiten Scheibe kann dabei auch auf einfache Weise aus Kunststoff erfolgen, weil die chirurgische Reinigungsvorrichtung als Wegwerfprodukt keine lange Haltbarkeit erfordert. Der Verschleiß ist also für die vorliegende Anwendung nicht von Bedeutung.

Bevorzugt hat die geneigte Oberfläche der Scheibe eine Neigung zwischen 5° und 45° zur Drehachse der Antriebswelle oder die geneigte Oberfläche der ersten Scheibe und/oder die geneigte Oberfläche der zweiten Scheibe haben eine Neigung zwischen 5° und 45° zur Drehachse der Antriebswelle.

Des Weiteren kann vorgesehen sein, dass mit dem Rückstellelement die Scheibe gegen die Antriebswelle gedrückt wird oder das hintere Ende des Rohrs gegen die Antriebsscheibe gedrückt wird oder die zweite Scheibe gegen die erste Scheibe gedrückt wird.

Besonders bevorzugt kann vorgesehen sein, dass das Getriebe eine erste Scheibe aufweist, die mit dem vorderen Ende der Antriebswelle verbunden ist oder die an dem vorderen Ende der Antriebswelle befestigt ist, wobei die erste Scheibe schräg zur Drehachse der Antriebswelle gestellt ist, und das Getriebe eine zweite Scheibe aufweist, wobei die zweite Schreibe mit dem hinteren Ende des Rohrs verbunden ist oder die zweite Schreibe an dem hinteren Ende des Rohrs befestigt ist, und wobei die zweite Scheibe schräg zur Längsachse des Rohrs oder schräg zur Drehachse der Antriebswelle gestellt ist und wobei die zweite Scheibe durch ein elastisches Rückstellelement gegen die erste Scheibe gedrückt wird.

Das bedeutet, dass bei einer Drehung der Antriebswelle die erste Scheibe rotiert und gegen die schräg zur Längsachse am hinteren Ende des Rohrs angeordnete zweite Scheibe gedrückt wird. Die zweite Scheibe kann nicht mit der ersten Scheibe rotieren, wenn durch eine Führung eine Drehbewegung des Rohrs verhindert wird. Auf Grund der Schrägstellung der ersten Scheibe wird die zweite Scheibe axial in Richtung des Bürstenkopfs bewegt. Der Betrag der axialen Bewegung ist durch den Abstand des entferntesten und des nächsten Punkts der ersten Scheibe in Bezug auf die Längsachse der Antriebswelle definiert. Durch die Führung ist nur eine axiale beziehungsweise lineare Bewegung des Rohrs möglich. Dadurch drückt die schräggestellte erste Scheibe bei ihrer Rotation gegen die nicht mitrotierende zweite Scheibe und presst diese periodisch in Richtung des Bürstenkopfs. Durch das Rückstellelement wird die zweite Scheibe und damit das Rohr nach erfolgter axialer beziehungsweise linearer Auslenkung in Richtung des Bürstenkopfs wieder zurück in Richtung der ersten Scheibe gedrückt. Dadurch entsteht bei jeder Umdrehung der ersten Scheibe eine axiale Vorwärts- und Rückwärtsbewegung der zweiten Scheibe und des Rohrs mit dem Bürstenkopf.

Des Weiteren kann vorgesehen sein, dass eine Führung im Gehäuse eine Drehbewegung des Rohrs gegen das Gehäuse verhindert, wobei vorzugsweise das Gehäuse oder eine Führung des Gehäuses die lineare Bewegung des Rohrs führt.

Hierdurch wird sichergestellt, dass sich das Rohr und damit der Bürstenkopf nicht ungewollt mit der Antriebswelle drehen können.

Ferner kann vorgesehen sein, dass der Bürstenkopf über eine lösbare Verbindung mit dem Rohr verbunden ist.

Hierdurch ist ein Aufsetzen eines für die jeweilige Anwendung geeigneten Bürstenkopfs möglich.

Auch kann vorgesehen sein, dass zwischen dem Bürstenkopf und dem Rohr oder in dem Rohr ein flexibel knickbarer Rohrabschnitt angeordnet ist, der ein Abknicken des Bürstenkopfs gegen die Längsachse des Rohrs um bis zu 45° zulässt, wobei bevorzugt der flexibel knickbare Rohrabschnitt als Muffe ausgebildet ist.

Damit können auch schwer zugängliche Bereiche mit dem Bürstenkopf zugänglich gemacht werden. Der flexibel knickbare Rohrabschnitt ist vorzugsweise außerhalb des Gehäuses angeordnet.

Des Weiteren kann vorgesehen sein, dass zumindest eine rohrförmige elastische Borste als eine der zumindest einen elastischen Borsten vorgesehen ist, wobei die zumindest eine rohrförmige elastische Borste innen hohl ist und mit dem Rohr flüssigkeitsdurchlässig verbunden ist, wobei vorzugsweise eine freie Öffnung der zumindest einen rohrförmigen elastischen Borste eine Ausspritzöffnung der zumindest einen Ausspritzöffnung ist.

Hierdurch kann die Spülflüssigkeit durch die zumindest eine rohrförmige elastische Borsten direkt an dem Ort appliziert werden, an der sich die mechanische Wirkung der zumindest einen rohrförmigen elastischen Borsten entfaltet. Hierdurch kann eine stärkere Reinigungswirkung erzielt werden und/oder der Volumenstrom der Spülflüssigkeit reduziert werden, was für bestimmte Anwendungen der chirurgischen Reinigungsvorrichtung vorteilhaft ist.

Es kann vorgesehen sein, dass die elastischen verformbaren Borsten hohl sind und Ausspritzöffnungen an den dem Bürstenkopf abgewandten Borstenenden besitzen.

Es kann auch vorgesehen sein, dass zumindest eine Nocke auf der Antriebswelle befestigt ist und ein elastisch verformbarer Schlauch an das hintere Ende des Rohrs angeschlossen ist, wobei der elastisch verformbare Schlauch derart im Wirkbereich der Nocken angeordnet ist, dass bei einer vollständigen 360°-Drehung der Antriebswelle um ihre Längsachse ein Spülflüssigkeitsstrom durch den elastisch verformbaren Schlauch mindestens einmal pro Umdrehung der Antriebswelle unterbrochen wird und dadurch pulsierende Spülflüssigkeitsstrahlen aus der zumindest einen Ausspritzöffnung austreten.

Hierdurch wird erreicht, dass ein Spülflüssigkeitsstrom, der durch die zumindest eine Ausspritzöffnung austritt, mindestens einmal pro Umdrehung der Antriebswelle unterbrochen wird und pulsierende Spülflüssigkeitsstrahlen entstehen. Hierdurch wird die Reinigungswirkung der chirurgischen Reinigungsvorrichtung verbessert.

Des Weiteren kann vorgesehen sein, dass die zumindest eine Ausspritzöffnung derart geformt und derart relativ zu den elastisch verformbaren Borsten angeordnet ist, dass ein durch die zumindest eine Ausspritzöffnung ausgestoßener Strahl der Spülflüssigkeit in den Wirkbereich der linear oszillierenden elastisch verformbaren Borsten trifft.

Der Wirkbereich der linear oszillierenden elastisch verformbaren Borsten ist der Bereich, den die freien Enden der elastisch verformbaren Borsten, die einer Verbindung der elastisch verformbaren Borsten zum Bürstenkopf gegenüberliegen, bei der linear oszillierenden Bewegung der elastisch verformbaren Borsten überstreichen. Das ist der Bereich der chirurgischen Reinigungsvorrichtung, der zur mechanischen Reinigung mit Hilfe der elastisch verformbaren Borsten vorgesehen ist.

Durch das Auftreffen des Strahls der Spülflüssigkeit im Wirkbereich der linear oszillierenden elastisch verformbaren Borsten wird eine besonders gute Reinigungswirkung erzielt, da die von den elastisch verformbaren Borsten mechanisch abgelösten Partikel von dem Strom der Spülflüssigkeit abtransportiert werden können.

Die der vorliegenden Erfindung zugrundeliegenden Aufgaben werden auch gelöst durch ein Verfahren zum Betreiben einer chirurgischen Reinigungsvorrichtung, wobei das Verfahren die folgenden Schritte aufweist:
A) Drehen einer Antriebswelle der chirurgischen Reinigungsvorrichtung in einem Gehäuse der chirurgischen Reinigungsvorrichtung;
B) Übersetzen der Drehung der Antriebswelle in eine lineare oszillierende Bewegung eines Rohrs und einem an einem vorderen Ende des Rohrs befestigten Bürstenkopfs mit einem Getriebe, wobei das Rohr linear beweglich in dem Gehäuse gelagert ist;
C) Pumpen einer Spülflüssigkeit durch das linear oszillierende Rohr zu zumindest einer Ausspritzöffnung im Bürstenkopf, wobei das Pumpen der Spülflüssigkeit mit einer Pumpe durchgeführt wird, wobei die Pumpe mit der sich drehenden Antriebswelle angetrieben wird; und
D) Einspritzen der aus der zumindest einen Ausspritzöffnung in den Wirkbereich der elastisch verformbaren Borsten.

Das Verfahren kann beispielsweise zur Reinigung einer gebrauchten explantierten Prothese verwendet werden, damit diese anschließend wiederverwendet werden kann. Es ist aber auch möglich, mit dem Verfahren andere neue und gebrauchte Implantate oder neue oder gebrauchte medizinische Instrumente vor deren Verwendung zu reinigen.

Insbesondere kann vorgesehen sein, dass das Verfahren nicht zu einer medizinischen oder therapeutischen Behandlung eines Patienten eingesetzt wird.

Bevorzugt ist bei erfindungsgemäßen Verfahren eine Anwendung nicht patentierbarer medizinischer Verfahren ausgeschlossen. Wie bereits ausgeführt, kann das Verfahren zur Reinigung von OP-Werkzeugen und explantierten Implantaten verwendet werden, die nicht mit dem Körper eines Patienten verbunden sind, so dass dann keine unmittelbar auf den Körper einwirkende medizinische Anwendung vorliegt.

Das Drehen der Antriebswelle erfolgt vorzugsweise motorgetrieben und besonders bevorzugt durch eine externe Antriebseinrichtung.

Bei erfindungsgemäßen Verfahren kann vorgesehen sein, dass das Verfahren mit einer der zuvor beschriebenen erfindungsgemäßen chirurgischen Reinigungsvorrichtung durchgeführt wird.

Das Verfahren hat dann die zu der chirurgischen Reinigungsvorrichtung geschilderten Vorteile.

Es kann auch vorgesehen sein, dass die Spülflüssigkeit ungepulst oder mit der sich drehenden Antriebswelle gepulst in den Wirkbereich der linear oszillierenden elastisch verformbaren Borsten gesprüht wird.

Hierdurch kann die sich drehende Antriebswelle nicht nur die lineare oszillierende Bewegung des Bürstenkopfs antreiben, sondern auch den Spülflüssigkeitsstrahl antreiben. Die chirurgische Reinigungsvorrichtung benötigt dann neben der Antriebseinheit zum Antreiben der Drehung der Antriebswelle nur noch ein Spülflüssigkeitsreservoir, aus dem die Pumpe die Spülflüssigkeit mit der zweiten Flüssigkeitsleitung absaugen kann.

Des Weiteren kann vorgesehen sein, dass vor Schritt A) die chirurgische Reinigungsvorrichtung, insbesondere das Rohr oder die Pumpe, mit einem Spülflüssigkeitsreservoir verbunden wird.

Hierdurch wird klargestellt, dass das erfindungsgemäße Verfahren mit Hilfe eines externen Reservoirs der Spülflüssigkeit durchgeführt werden kann.

Ferner kann vorgesehen sein, dass die gebrauchte Spülflüssigkeit aus dem Bereich der Borsten zumindest teilweise mit einer Absaugeinrichtung abgesaugt wird, wobei bevorzugt nach der Absaugung mit einem Abscheider feste Partikel der gebrauchten Spülflüssigkeit von den flüssigen Bestandteilen getrennt werden.

Hierdurch kann die Reinigungswirkung des Verfahrens erhöht werden und eine Kontamination der Umgebung vermindert werden.

Bevorzugt kann auch vorgesehen sein, dass vor Schritt A) die chirurgische Reinigungsvorrichtung an eine Antriebseinheit angeschlossen wird, wobei die Drehung der Antriebswelle in den Schritten A) bis D) mit der Antriebseinheit angetrieben wird, wobei bevorzugt die Antriebseinheit an ein hinteres Ende der Antriebswelle angeschlossen wird.

Hierdurch wird klargestellt, dass eine externe Antriebseinheit zum Antreiben und Drehen der Antriebswelle der chirurgischen Reinigungsvorrichtung verwendet wird beziehungsweise verwendet werden kann.

Es kann des Weiteren vorgesehen sein, dass in Schritt B) eine gegen die Drehachse der Antriebswelle geneigte erste Scheibe, die an einem vorderen Ende der Antriebswelle befestigt ist, auf dem hinteren Ende des linear beweglichen Rohrs oder auf einer gegen die Drehachse der Antriebswelle geneigte zweite Scheibe abrollt, wobei bevorzugt die zweite Scheibe mit einem Rückstellelement gegen die erste Scheibe gedrückt wird.

Hierdurch kann auf einfache Weise ein Getriebe realisiert werden, mit dem die Drehung der Antriebswelle in eine linear oszillierende Bewegung übersetzt werden kann.

Der Erfindung liegt die überraschende Erkenntnis zugrunde, dass eine chirurgische Reinigungsvorrichtung durch den Antrieb einer einzelnen sich drehenden Antriebswelle sowohl zum Betreiben einer linear oszillierenden Bürste mit elastisch verformbaren Borsten verwendet werden kann und die Drehung der Antriebswelle auch gleichzeitig zum Erzeugen eines gepulsten oder auch ungepulsten Stroms einer Spülflüssigkeit im Wirkbereich der elastisch verformbaren Borsten durch Antreiben einer Pumpe mit der Antriebswelle genutzt werden kann. Dabei kann die Antriebswelle mit einer im OP üblichen Antriebseinheit antreibbar sein und die chirurgische Reinigungsvorrichtung derart einfach und kostengünstig im Aufbau ausgeführt werden, dass die chirurgische Reinigungsvorrichtung als kostengünstiges Wegwerfprodukt gefertigt werden kann, die eine hygienische Entsorgung nach einmaligem Gebrauch ermöglicht. Insbesondere ist eine sehr weitgehende und sogar vollständige Fertigung der chirurgischen Reinigungsvorrichtung aus Kunststoff möglich, der Rückstandsfrei verbrannt werden kann. Die chirurgische Reinigungsvorrichtung kann hierbei als einfacher Aufsatz zur Befestigung an einer externen Antriebseinheit aufgebaut werden. Der erfindungsgemäße Aufbau ermöglicht dabei, dass die chirurgische Reinigungsvorrichtung sehr weitgehend oder vollständig aus Kunststoff herstellbar ist, wodurch eine hygienische Entsorgung durch Verbrennung mit andere OP-Abfällen ermöglicht wird.

Gleichzeitig wird der Arbeitsablauf bei der Anwendung der chirurgischen Reinigungsvorrichtung dadurch vereinfacht, dass die Schritte des mechanischen Entfernens von Rückständen und des Spülens der gereinigten Oberfläche mit Spülflüssigkeit zur gleichen Zeit erfolgen kann. Dabei wird die Reinigungswirkung erhöht. Insbesondere bei der Verwendung einer flexiblen Antriebswellenverlängerung kann die externe Antriebseinheit derart weit von dem zu reinigenden Areal angeordnet werden, dass eine Verschmutzung der Antriebseinheit verhindert werden kann. Durch diese Maßnahmen kann ein aufwendiges Reinigen der chirurgischen Reinigungsvorrichtung und der externen Antriebseinheit entfallen und dadurch das Risiko einer nicht ausreichenden Reinigung und dadurch das Risiko einer gefährlichen Infektion bei erneuter Anwendung der chirurgischen Reinigungsvorrichtung und der Antriebseinheit nach erfolgter Reinigung reduziert beziehungsweise ausgeschlossen werden.

Die erfindungsgemäße chirurgische Reinigungsvorrichtung kann durch eine OP-übliche Antriebsvorrichtung angetrieben werden. Das Reinigen mit der chirurgischen Reinigungsvorrichtung erfolgt durch oszillierendes Bürsten mit elastisch verformbaren Borsten und durch ein oder mehrere nicht gepulste oder gepulste Spülflüssigkeitsstrahlen.

Die Reinigung von Gewebearealen erfolgt erfindungsgemäß mechanisch durch Einwirkung der elastisch verformbaren Borsten und gleichzeitig durch ein oder mehrere nicht gepulste oder gepulste Spülflüssigkeitsstrahlen, wobei die Spülflüssigkeit von außen durch eine Schlauchzuleitung beziehungsweise eine Flüssigkeitsleitung in die Vorrichtung geleitet werden kann. Die Spülflüssigkeit wird dabei erfindungsgemäß durch die in der chirurgischen Reinigungsvorrichtung enthaltenen Pumpe angesaugt und anschließend in Form von Sprühstrahlen wieder ausgestoßen. Diese Spülflüssigkeitsstrahlen strömen entweder ungepulst oder gepulst aus der chirurgischen Reinigungsvorrichtung aus. Weiterhin ist es möglich, die chirurgische Reinigungsvorrichtung mit Hilfe einer Absaugeinrichtung derart auszugestalten, dass die gebrauchte Spülflüssigkeit nach erfolgter Reinigung mit Hilfe der chirurgischen Reinigungsvorrichtung aus dem gereinigten Gewebeareal zumindest teilweise wieder entfernt werden kann.

Die gleichzeitige Anwendung von linear oszillierenden elastisch verformbaren Borsten und zumindest eines Sprühstrahls einer Spülflüssigkeit und der erfindungsgemäße Aufbau der chirurgischen Reinigungsvorrichtung ermöglichen, dass synchron zum Antrieb der linear oszillierenden elastisch verformbaren Borsten gleichzeitig Spülflüssigkeit ausgesprüht werden kann und gegebenenfalls auch die gebrauchte Spülflüssigkeit wieder angesaugt werden kann, so dass ein Reinigungseffekt durch die gleichzeitige Einwirkung der linear oszillierenden elastisch verformbaren Borsten zusammen mit Spülflüssigkeitsstrahlen erfolgen kann. Durch die linear oszillierenden elastisch verformbaren Borsten mechanisch abgetragene Verunreinigungen werden durch die gleichzeitig einwirkenden Spülflüssigkeitsstrahlen weggespült. Dadurch wird ein erneutes Anhaften der Verunreinigungen an den gereinigten Oberflächen verhindert.

Elektrische Antriebseinheiten für chirurgische Werkzeuge sind weltweit in der Chirurgie und Orthopädie üblich. Diese werden zum Antrieb von oszillierenden Sägen und zum Bohren genutzt. Als Energiequelle für solche Antriebseinheiten werden üblicherweise Akkumulatoren eingesetzt. Die erfindungsgemäße chirurgische Reinigungsvorrichtung kann problemlos mit dem hinteren Ende der Antriebswelle über ein Bohrfutter mit der externen Antriebseinheit verbunden werden. Der medizinische Anwender hält mit einer Hand die Antriebseinheit und betätigt mit der anderen Hand die Antriebsvorrichtung. Durch das Festhalten der chirurgischen Reinigungsvorrichtung wird ein Mitdrehen der chirurgischen Reinigungsvorrichtung mit der Antriebswelle verhindert. Bei der Verwendung einer Antriebswellenverlängerung kann die externe Antriebseinheit auch einfach an einem entfernten Ort aufgestellt und fixiert werden und der Anwender muss nur die chirurgische Reinigungsvorrichtung halten.

Eine beispielhafte erfindungsgemäße chirurgische Reinigungsvorrichtung ist zusammengesetzt aus
a) einem Gehäuse,
b) einer frei drehbaren Antriebswelle, die mit einem hinteren Wellenende aus dem Gehäuse herausragt, das zur Verbindung mit einer Antriebsvorrichtung bestimmt ist,
c) einem vorderen Wellenende, das im Inneren des Gehäuses angeordnet ist,
d) einer Flüssigkeitszuleitung, die mit einem Flüssigkeitsreservoir verbunden ist,
e) einer Pumpe, die im Inneren des Gehäuses angeordnet ist und mit der Antriebswelle verbunden ist, wobei die Pumpe flüssigkeitsdurchlässig mit der Flüssigkeitszuleitung verbunden ist,
f) einem Rohr, das axial beweglich im Gehäuse angeordnet ist, das ein hinteres Rohrende und ein vorderes Rohrende besitzt, und das aus dem Gehäuse herausragt, wobei das Rohr mit der Pumpe flüssigkeitsdurchlässig verbunden ist,
g) einem Hohlkörper als Bürstenkopf, der mit dem vorderen Rohrende flüssigkeitsdurchlässig verbunden ist, der auf mindestens einer Seite elastische Borsten und Ausspritzöffnungen besitzt, wobei die Ausspritzöffnungen den Innenraum des Hohlkörpers mit der Umgebung verbinden,
h) einem elastischen Rückstellelement, welches das Rohr in Richtung des vorderen Wellenendes drückt,
i) einem Getriebe, das zwischen dem vorderen Wellenende und dem Rohr angeordnet ist, das die Drehbewegung der Antriebswelle in eine oszillierende lineare Bewegung des Rohrs umformt, wobei
j) bei der Drehbewegung der Antriebswelle eine oszillierende, axiale, lineare Bewegung des Rohrs mit dem Hohlkörper mit den elastischen Borsten und gleichzeitig ein Ansaugen der Spülflüssigkeit und ein Herausspritzen der Spülflüssigkeit aus den Ausspritzöffnungen des Hohlkörpers mit Hilfe der Pumpe erfolgt.

Ein beispielhaftes erfindungsgemäßes Verfahren zur Reinigung mit einer chirurgischen Reinigungsvorrichtung kann die folgenden Schritte aufweisen:
a) Verbinden der chirurgischen Reinigungsvorrichtung mit einer Antriebseinheit,
b) Verbinden der Reinigungsvorrichtung mit einem Spülflüssigkeitsreservoir,
c) Drehen der Antriebswelle,
d) Erzeugen einer linearen oszillierenden Bewegung eines Rohrs mit einem daran befestigten Bürstenkopf in Form eines Hohlkörpers und mit am Bürstenkopf befestigten elastisch verformbaren Borsten, wobei die lineare oszillierende Bewegung mit Hilfe eines Getriebes von der Drehung der Antriebswelle angetrieben wird,
e) Antreiben einer Pumpe durch die Drehbewegung der Antriebswelle,
f) Pumpen der Spülflüssigkeit mit der Pumpe, wobei die Spülflüssigkeit gepulst oder ungepulst aus zumindest einer Spritzöffnungen austritt.

In einer Variante des Verfahrens wird die gebrauchte Spülflüssigkeit mit einem äußeren Rohr und einer externen Absaugvorrichtung abgesaugt.

Im Folgenden werden weitere Ausführungsbeispiele der Erfindung anhand von achtzehn schematisch dargestellten Figuren erläutert, ohne jedoch dabei die Erfindung zu beschränken. Dabei zeigt:
Figur 1: eine schematische perspektivische Ansicht einer beispielhaften ersten erfindungsgemäßen chirurgischen Reinigungsvorrichtung;
Figur 2: eine schematische perspektivische Teilschnitt-Ansicht der ersten erfindungsgemäßen chirurgischen Reinigungsvorrichtung nach Figur 1 mit in Längsrichtung geschnittenem und geöffnetem Gehäuse;
Figur 3: eine schematische perspektivische Querschnittansicht in Längsrichtung der ersten erfindungsgemäßen chirurgischen Reinigungsvorrichtung nach den Figuren 1 und 2 zur Veranschaulichung des inneren Aufbaus;
Figur 4: eine schematische perspektivische Querschnittansicht in horizontaler Richtung der ersten erfindungsgemäßen chirurgischen Reinigungsvorrichtung nach den Figuren 1 bis 3 zur Veranschaulichung des inneren Aufbaus;
Figur 5: eine weitere schematische perspektivische Querschnittansicht in horizontaler Richtung der ersten erfindungsgemäßen chirurgischen Reinigungsvorrichtung nach den Figuren 1 bis 4 aus einem im Vergleich zu Figur 4 gegenüberliegenden Blickwinkel;
Figur 6: eine schematische Teilschnitt-Ansicht einer Pumpe der ersten erfindungsgemäßen chirurgische Reinigungsvorrichtung;
Figur 7: eine schematische seitliche Teil-Querschnittansicht in Längsrichtung der ersten erfindungsgemäßen chirurgischen Reinigungsvorrichtung nach den Figuren 1 bis 6 bei geöffnetem Gehäuse und geöffnetem Bürstenkopf;
Figur 8: eine schematische seitliche Querschnittansicht in Längsrichtung der ersten erfindungsgemäßen chirurgischen Reinigungsvorrichtung nach den Figuren 1 bis 7;
Figur 9: eine schematische Querschnittansicht auf einen Schnitt in horizontaler Richtung der ersten erfindungsgemäßen chirurgischen Reinigungsvorrichtung nach den Figuren 1 bis 8;
Figur 10: eine schematische seitliche Querschnittansicht in Längsrichtung der ersten erfindungsgemäßen chirurgischen Reinigungsvorrichtung nach den Figuren 1 bis 9 während des Betriebs;
Figur 11: eine schematische seitliche Querschnittansicht in Längsrichtung der ersten erfindungsgemäßen chirurgischen Reinigungsvorrichtung nach den Figuren 1 bis 10 während des Betriebs mit noch vorne (links) ausgelenktem Bürstenkopf;
Figur 12: eine schematische perspektivische seitliche Ansicht einer zweiten erfindungsgemäßen chirurgischen Reinigungsvorrichtung mit einem knickbaren Rohr;
Figur 13: eine schematische perspektivische seitliche Ansicht der zweiten erfindungsgemäßen chirurgischen Reinigungsvorrichtung nach Figur 12 mit geknicktem knickbaren Rohr;
Figur 14: eine schematische perspektivische seitliche Ansicht einer dritten erfindungsgemäßen chirurgischen Reinigungsvorrichtung mit gekrümmtem Bürstenkopf;
Figur 15: eine schematische perspektivische Ansicht eines Bürstenkopfs für eine vierte erfindungsgemäße chirurgische Reinigungsvorrichtung mit hohlen Borsten;
Figur 16: eine schematische Querschnittansicht des Bürstenkopfs für die vierte erfindungsgemäße chirurgische Reinigungsvorrichtung nach Figur 15;
Figur 17: eine schematische perspektivische Ansicht einer fünften erfindungsgemäßen chirurgischen Reinigungsvorrichtung mit einer Absaugeinrichtung; und
Figur 18: eine schematische perspektivische Ansicht (links oben), eine schematische perspektivische Querschnittansicht (Mitte) und eine schematische Querschnittansicht (rechts unten) auf eine Antriebswellenverlängerung für eine erfindungsgemäße chirurgische Reinigungsvorrichtung.

In den Figuren und in der folgenden Beschreibung zu den anhand der Figuren erläuterten Ausführungsbeispielen der vorliegenden Erfindung werden teilweise für unterschiedliche Ausführungsbeispiele und für unterschiedliche Einzelteile die gleichen Bezugszeichen bei gleichen oder gleichartigen Teilen verwendet, um die Vergleichbarkeit der Ausführungsbeispiele und die Lesbarkeit zu vereinfachen.

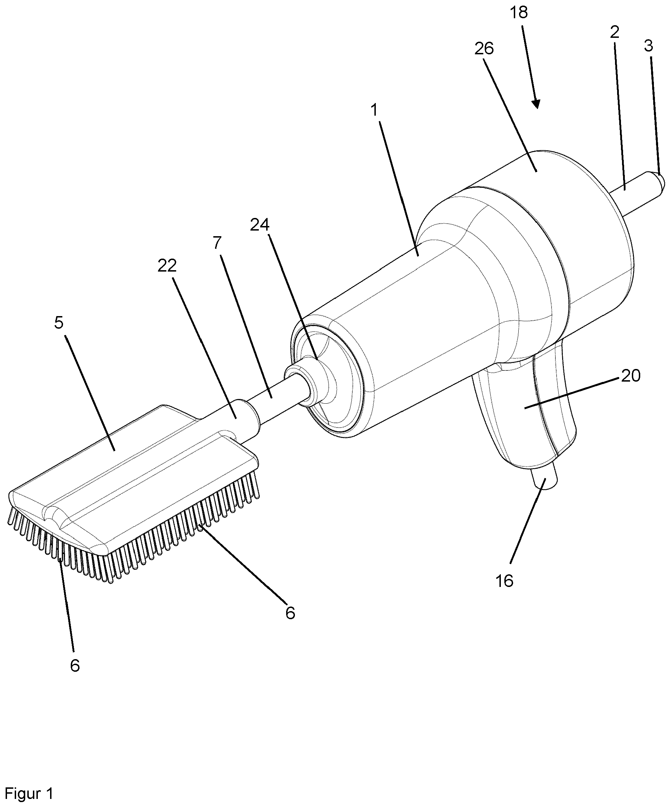

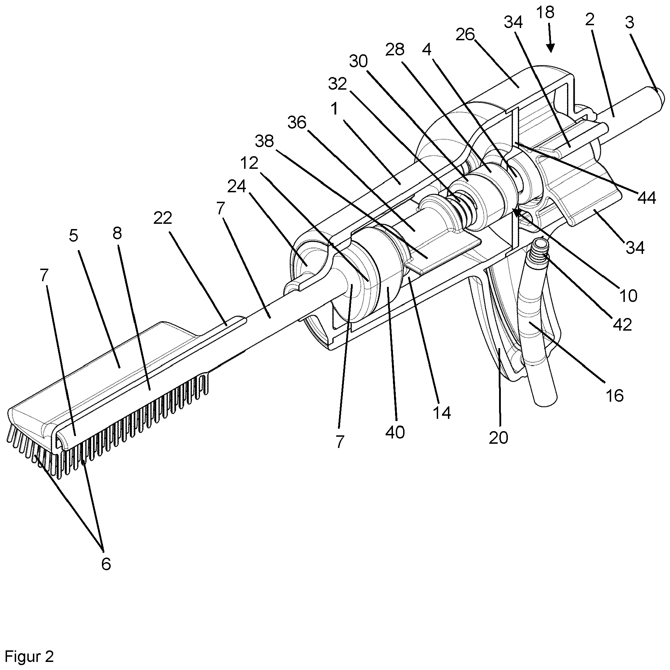

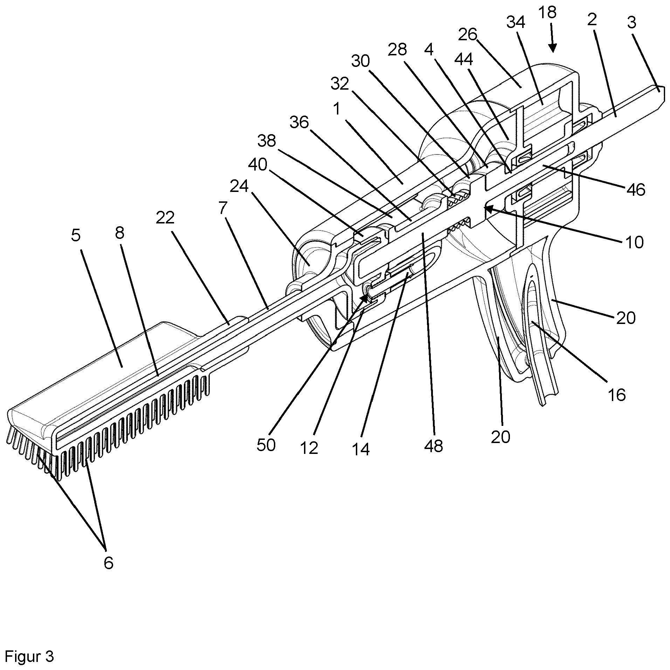

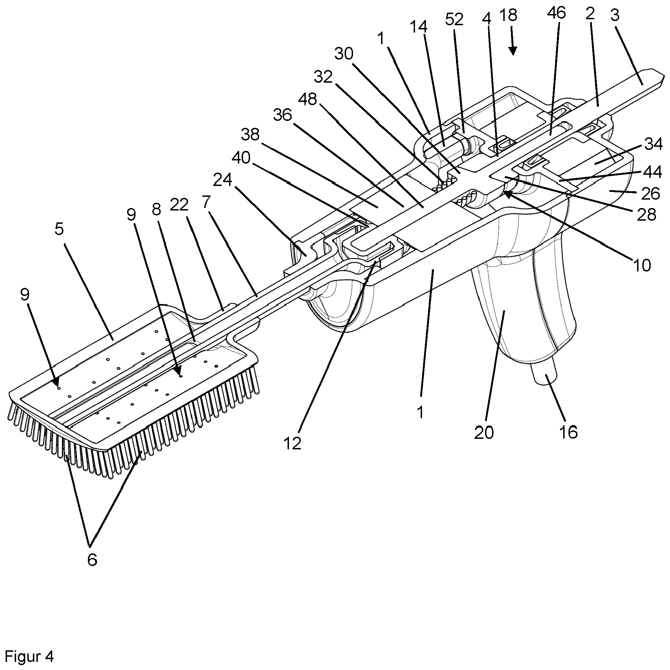

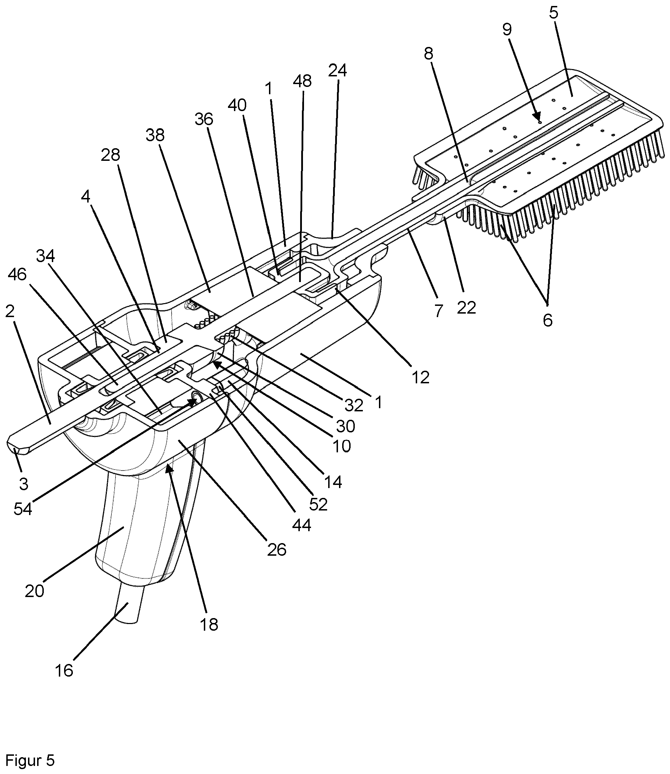

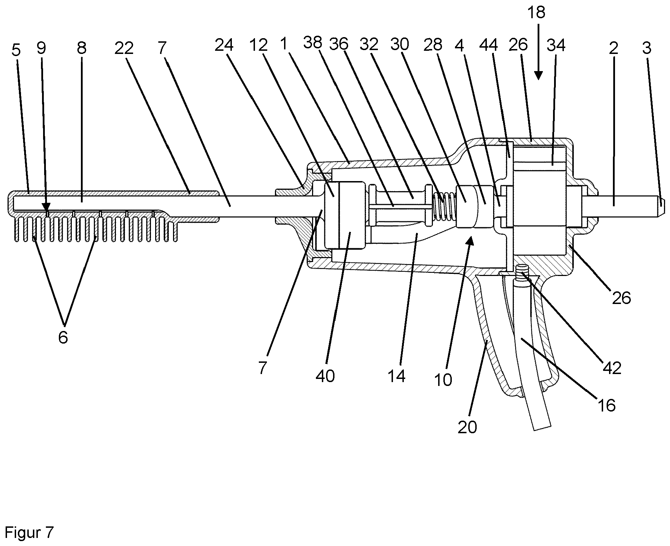

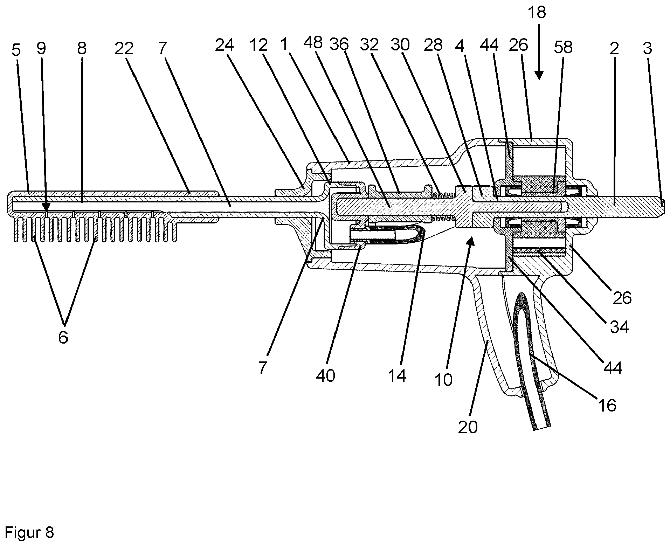

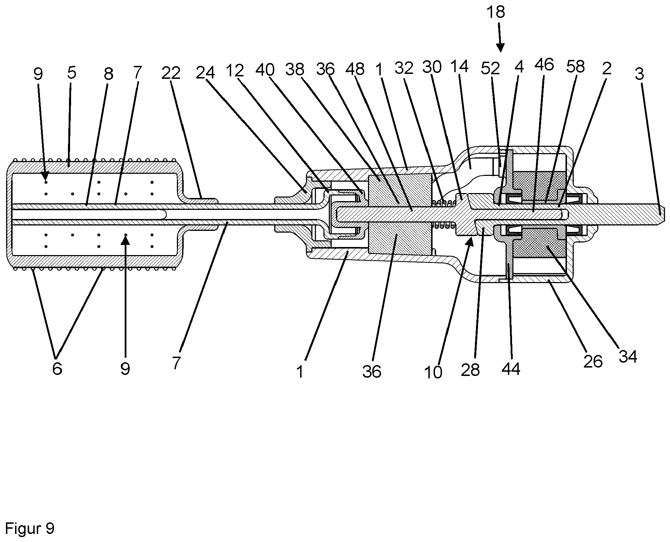

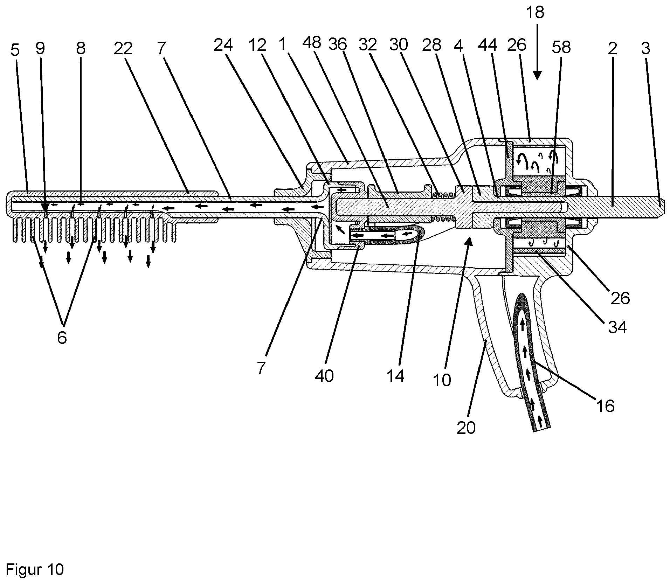

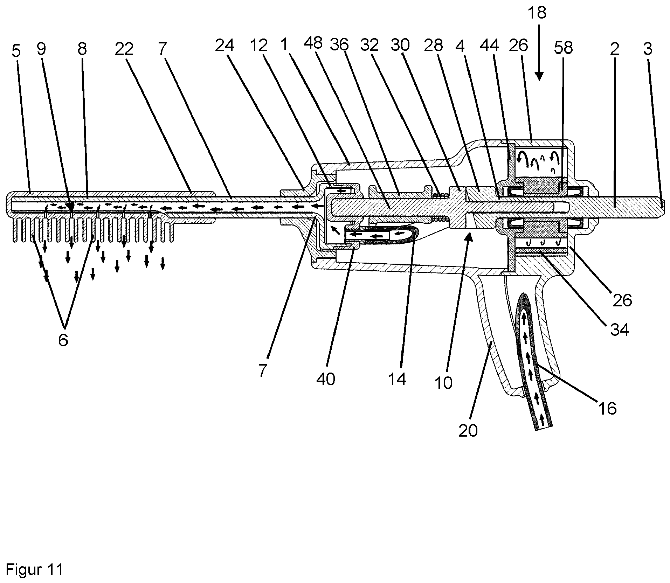

Figur 1 zeigt eine perspektivische Außenansicht einer beispielhaften ersten erfindungsgemäßen chirurgischen Reinigungsvorrichtung. In den Figuren 2 bis 11 sind weitere Ansichten, Querschnittansichten und Detailansichten der ersten chirurgischen Reinigungsvorrichtung und ihrer Teile gezeigt, wobei die Figuren 10 und 11 den Betrieb der ersten erfindungsgemäßen chirurgischen Reinigungsvorrichtung als Querschnittansichten zeigen. Die erste chirurgische Reinigungsvorrichtung weist ein Gehäuse 1 auf, das die chirurgische Reinigungsvorrichtung in Teilen nach außen abschließt und in einem hinteren Bereich als Griffstück 20 zum Halten der chirurgischen Reinigungsvorrichtung verwendet werden kann.

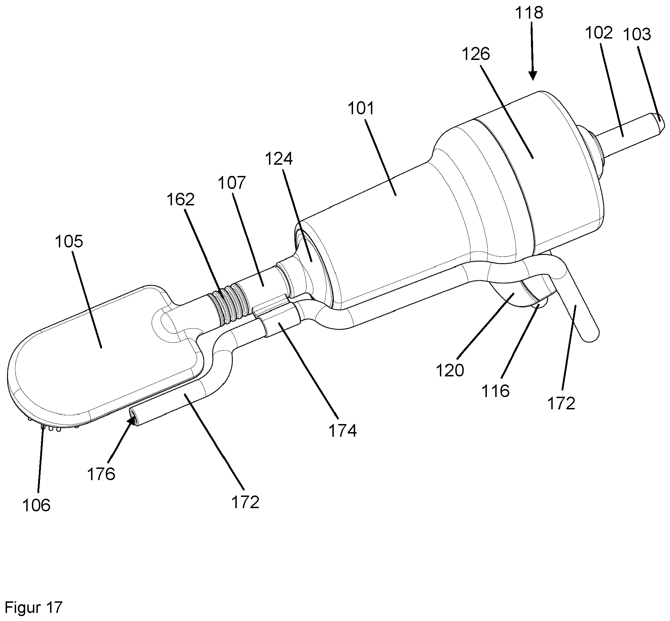

Im Inneren des Gehäuses 1 befindet sich eine darin drehbar gelagerte Antriebswelle 2. Die Antriebswelle 2 weist ein hinteres Ende 3 auf, das direkt oder über eine Antriebswellenverlängerung 200 (siehe Figur 17) an eine externe Antriebseinheit (nicht gezeigt) angeschlossen oder anschließbar ist. Die externe Antriebseinheit kann beispielsweise ein im OP-Bereich üblicher Mehrzweck-Antrieb sein oder eine Bohrmaschine oder ähnliches. Dem hinteren Ende 3 gegenüberliegend weist die Antriebswelle 2 ein vorderes Ende 4 auf. An der Vorderseite der chirurgischen Reinigungsvorrichtung ist ein Bürstenkopf 5 angeordnet, wobei an dem Bürstenkopf 5 eine Vielzahl elastisch verformbarer Borsten 6 befestigt sein können.

Der Bürstenkopf 5 ist an einem vorderen Ende 8 eines Rohrs 7 befestigt. Das Rohr 7 kann auch zur Einleitung von Spülflüssigkeit in den Bürstenkopf 5 dienen. Der Bürstenkopf 5 kann als Hohlkörper geformt sein oder in seinem Inneren Leitungen zur Leitung der Spülflüssigkeit enthalten. Auf einer Unterseite des Bürstenkopfs 5 können mehrere Ausspritzöffnungen 9 vorgesehen sein, die flüssigkeitsleitend mit dem Rohr 7 verbunden sein können. Hierzu können die Ausspritzöffnungen 9 die Umgebung des Bürstenkopfs 5 mit einem Innenraum des Bürstenkopfs 5 verbinden, wobei der Innenraum des Hohlkörpers beziehungsweise des Bürstenkopfs 5 mit dem Rohr 7 flüssigkeitsleitend verbunden ist. Bevorzugt mündet das Rohr 7 in den Innenraum des Hohlkörpers beziehungsweise des Bürstenkopfs 5. Die Ausspritzöffnungen 9 münden im Bereich der elastische verformbaren Borsten 6 in deren Wirkbereich, das heißt in den Bereich, den die elastisch verformbaren Borsten 6 bei einer linear oszillierenden Bewegung des Bürstenkopfs 5 überstreichen.

Zwischen dem Rohr 7 und der Antriebswelle 2 ist ein Getriebe 10 angeordnet, das eine Drehung der Antriebswelle 2 in eine lineare oszillierende Bewegung des Rohrs 7 mit dem Bürstenkopf 5 übersetzt. Hierzu kann das Getriebe 10 auf ein hinteres Ende 12 des Rohrs 7 einwirken. Das hintere Ende 12 des Rohrs 7 kann über eine Kappe 40 mit einer ersten Flüssigkeitsleitung 14 verbunden sein. Hierzu kann die erste Flüssigkeitsleitung 14 über eine Einmündung 50 in der Kappe 40 in das Rohr 7 einmünden. Durch die erste Flüssigkeitsleitung 14 kann Spülflüssigkeit in das Rohr 7 eingespeist werden. Die Spülflüssigkeit kann anschließend durch das Rohr 7 und durch den Bürstenkopf 5 zu den Ausspritzöffnungen 9 fließen und von dort in den Wirkbereich der Borsten 6 gespritzt werden. Die erste Flüssigkeitsleitung 14 kann ein flexibler Schlauch sein, der auf einer Seite mit der Verschlusskappe 40 am hinteren Ende 12 des Rohrs 7 und auf der anderen Seite mit dem Gehäuse 1 verbunden ist. Der Schlauch kann die lineare oszillierende Bewegung des Rohrs 7 gegen das Gehäuse 1 durch eine Verformung des Schlauchs bei der Bewegung des Rohrs 7 ausgleichen.

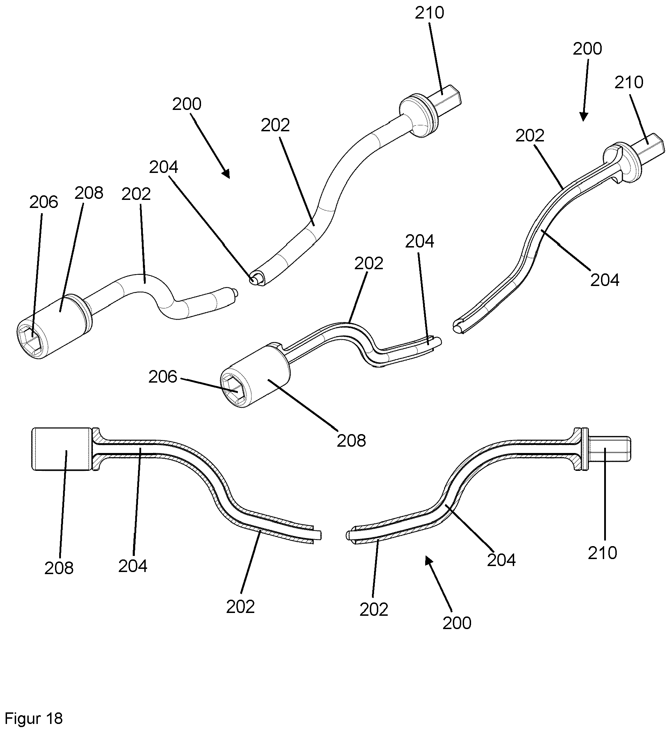

Die Antriebswelle 2 kann am hinteren Ende 3 als Anschluss in Form eines Sechskants oder einer anderen Mehrkanntstange ausgebildet sein. An das hintere Ende 3 beziehungsweise den Anschluss kann ein Spannfutter oder ein passender Gegenanschluss der externen Antriebseinrichtung angeschlossen sein oder werden. Eine Antriebswellenverlängerung 200 (siehe Figur 18) kann zwischen das hintere Ende 3 beziehungsweise den Anschluss und den Gegenanschluss der externen Antriebseinrichtung angeschlossen werden.

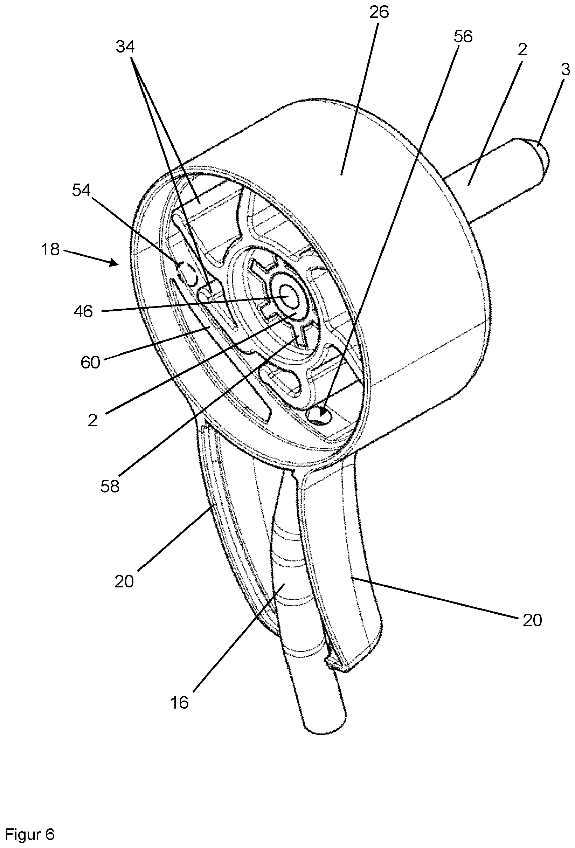

Die chirurgische Reinigungsvorrichtung kann eine Pumpe 18 aufweisen, die mit der Antriebswelle 2 im Bereich des hinteren Endes 3 der Antriebswelle 2 verbunden sein kann. Die Pumpe 18 kann über die Antriebswelle 2 angetrieben werden und kann zu diesem Zweck mit der Antriebswelle 2 verbunden sein. Die Pumpe 18 kann bevorzugt als Impellerpumpe ausgeführt sein und ist in Figur 6 im Detail gezeigt. Die Pumpe 18 ist an eine zweite Flüssigkeitsleitung 16 zum Zuführen einer Flüssigkeit in die Pumpe 18 und an die erste Flüssigkeitsleitung 14 zum Ableiten der Flüssigkeit aus der Pumpe 18 in das Rohr 7 angeschlossen. Anstatt der Impellerpumpe kann auch eine andere Pumpe verwendet werden. Die Impellerpumpe ist jedoch aufgrund ihres einfachen Aufbaus und aufgrund der Tatsache, dass die Impellerpumpe bei einem Stillstand der Impellerpumpe die zweite Flüssigkeitsleitung 16 von der ersten Flüssigkeitsleitung 14 physikalisch trennt, besonders bevorzugt.

Das Gehäuse 1 kann an seiner Unterseite als Griffstück 20 geformt sein. Das Griffstück 20 kann zur einfachen Handhabung der chirurgischen Reinigungsvorrichtung nach Art eines Pistolengriffs ausgeführt sein. Der Bürstenkopf 5 kann einen rohrförmigen Stutzen 22 aufweisen, mit dem der Bürstenkopf 5 flüssigkeitsdicht auf das Rohr 7 gesteckt und dort befestigt werden kann. Hierdurch kann es möglich sein, den Bürstenkopf 5 im Bedarfsfall einfach auszutauschen. Das Gehäuse 1 kann an seiner Vorderseite mit einem vorderen Verschluss 24 verschlossen sein. Dies erleichtert den Zusammenbau der chirurgischen Reinigungsvorrichtung. Der vordere Verschluss 24 kann zur linear beweglichen Lagerung des Rohrs 7 gegen das Gehäuse 1 genutzt werden.

Die Pumpe 18 kann ein Pumpengehäuse 26 aus Kunststoff aufweisen. Das Pumpengehäuse 26 kann außen einen Teil des Gehäuses 1 bilden. Die Antriebswelle 2 kann über Dichtungen abgedichtet durch das Pumpengehäuse 26 geführt und von dem Pumpengehäuse 26 gelagert sein.

Am Pumpengehäuse 26 kann ein Anschluss zum Verbinden der zweiten Flüssigkeitsleitung 16 mit einer Zuleitung von einer Flüssigkeitsquelle (nicht gezeigt) ausgebildet sein. Das Pumpengehäuse 26 kann auf seiner Vorderseite mit einer Frontplatte 44 verschlossen sein. Die Antriebswelle 2 kann über eine Lagerung in die Pumpe 18 geführt sein. An der Antriebswelle 2 können mehrere Impellerflügel 34 der Pumpe 18 befestigt sein. Die Impellerflügel 34 können über eine Aufnahme 58 an der Antriebswelle 2 befestigt sein. Die Impellerflügel 34 können senkrecht zur radialen Richtung elastisch verformbar sein und innen an dem Pumpengehäuse 26 anliegen. Durch die Drehung und eine in den Pumpenraum der Pumpe 18 vorstehende Wandung 60, erzeugt die Pumpe 18 dann bei der Drehung der Impellerflügel 34 eine Pumpwirkung.

In der Pumpe 18 kann also ein Impeller mit mehreren Impellerflügeln 34 angeordnet sein. Der Impeller kann auf der Antriebswelle 2 befestigt sein und sich mit der Antriebswelle 2 drehen. Hierzu kann die Aufnahme 58 auf der Antriebswelle 2 befestigt sein, die mit dem Impeller kraftschlüssig verbunden ist. Durch die Drehung des Impellers mit der Antriebswelle 2 kann dann die Pumpe 18 betrieben werden. Auf diese Weise erfolgt gleichzeitig mit der linearen Oszillation der elastisch verformbaren Borsten 6 das Pumpen einer Spülflüssigkeit mit der Pumpe 18 und damit das Ausstoßen eines Flüssigkeitsstrahls der Spülflüssigkeit in den Wirkbereich der elastisch verformbaren Borsten 6 (siehe die Pfeile in den Figuren 10 und 11).

Das Getriebe 10 kann eine erste Scheibe 28 in Form einer Keilscheibe aufweisen und kann eine zweite Scheibe 30 in Form einer Keilscheibe aufweisen. Die erste Scheibe 28 kann ebenso wie die zweite Scheibe 30 eine gegen die Drehachse der Antriebswelle 2 und gegen die lineare Bewegungsrichtung des Rohrs 7 geneigte Oberfläche aufweisen, wobei die geneigte Oberfläche der ersten Scheibe 28 an der geneigten Oberfläche der zweiten Scheibe 30 anliegt. Hierzu kann die zweite Scheibe 30 mit einem Rückstellelement 32 gegen die erste Scheibe 28 gedrückt werden. Das Rückstellelement 32 kann beispielsweise eine Druckfeder beziehungsweise eine Spiralfeder sein.

Die erste Scheibe 28 kann an dem vorderen Ende 4 der Antriebswelle 2 befestigt sein, so dass sich die erste Scheibe 28 mit der Antriebswelle 2 dreht. Die zweite Scheibe 30 kann über einen vorderen Schaft 48 mit dem Rohr 7 fest verbunden sein. Hierzu kann der vordere Schaft 48 in einer Verschlusskappe 40 an dem hinteren Ende 12 des Rohrs 7 befestigt sein. Die Verschlusskappe 40 kann das Rohr 7 am hinteren Ende 12 fluiddicht verschließen.

Der vordere Schaft 48 kann mit Hilfe eines Lagers 36 linear beweglich aber nicht drehbar gegen das Gehäuse 1 gelagert sein. Hierzu kann das Lager 36 über Flügel 38 mit dem Gehäuse 1 fest verbunden sein. Auf diese Weise kann verhindert werden, dass sich der vordere Schaft 48 und die zweite Scheibe 30 gegen das Gehäuse 1 drehen kann. Durch die Änderung der Neigung der ersten Scheibe 28 gegen die Neigung der zweiten Scheibe 30 bei der Drehung der Antriebswelle 2 wird mit dem Getriebe 10 die Drehung der ersten Scheibe 28 in eine oszillierende lineare Bewegung der zweiten Scheibe 30 übersetzt und dadurch die lineare oszillierende Bewegung des Bürstenkopfs 5 angetrieben (siehe Figuren 10 und 11). Gleichzeitig kann die Drehung der Antriebswelle 2 zum Antreiben der Pumpe 18 verwendet werden. Das Rückstellelement 32 kann sich an dem Lager 36 und an einer Vorderseite der zweiten Scheibe 30 abstützen.

An der Rückseite der zweiten Scheibe 30 kann ein hinterer Schaft 46 angeordnet sein. Der hintere Schaft 46 kann gleitend und linear beweglich in einer passenden Vertiefung der Antriebswelle 2 angeordnet sein. Dadurch wird die zweite Scheibe 30 passend zur ersten Scheibe 28 ausgerichtet.

Um einen pulsierenden Strom der Spülflüssigkeit zu erzeugen, kann eine Blende mit mindestens einer Ausnehmung vorgesehen sein, die bei jeder Drehung der ersten Scheibe 28 die erste Flüssigkeitsleitung 14 zumindest einmal zusammenpresst. Die Blende kann Bereiche mit unterschiedlich großen Radien aufweisen, so dass bei einer vollen Drehung der Antriebswelle 2 die Ausspritzöffnungen 9 beziehungsweise die erste Flüssigkeitsleitung 14 davor zeitweise geöffnet sind und zeitweise verschlossen sind. Hierdurch wird ein pulsierender Ausstoß der Spülflüssigkeit erzeugt, der mit der Umdrehungsgeschwindigkeit der Antriebswelle 2 getaktet ist. Gleichzeitig mit dem Antrieb der linear oszillierenden Bewegung der elastisch verformbaren Borsten 6 und dem Ausstoß der Spülflüssigkeit kann also auch ein pulsierender Betrieb des Flüssigkeitsstroms der Spülflüssigkeit über die Drehung der Antriebswelle 2 erzeugt werden.

Die erste Flüssigkeitsleitung 14 kann auf der der Verschlusskappe 40 gegenüberliegenden Seite über einen Anschluss 52 für die erste Flüssigkeitsleitung 14 an die Pumpe 18 mit der Pumpe 18 verbunden sein. Mit der Pumpe 18 kann die Spülflüssigkeit durch eine Einmündung 54 in die erste Flüssigkeitsleitung 14 gedrückt beziehungsweise gepumpt werden.

Im Bereich einer Einmündung 56 für die Spülflüssigkeit in die Pumpe 18 (siehe Figur 6) kann zum Anschließen der zweiten Flüssigkeitsleitung 16 ein Innengewinde zur Befestigung eines Außengewindes 42 der zweiten Flüssigkeitsleitung 16 angeordnet sein. Die Pumpe 18 kann über die zweite Flüssigkeitsleitung 16 mit einem Spülflüssigkeitsreservoir (nicht gezeigt) der Spülflüssigkeit verbunden werden. Die chirurgische Reinigungsvorrichtung kann betrieben werden, indem die chirurgische Reinigungsvorrichtung mit dem hinteren Ende 3 der Antriebswelle 2 direkt oder über eine Antriebswellenverlängerung 200 mit der externen Antriebseinheit verbunden wird. Die externe Antriebseinheit treibt die Antriebswelle 2 an. Durch die Drehung der Antriebswelle 2 kann die Pumpe 18 betrieben werden und gleichzeitig der mit den elastisch verformbaren Borsten 6 aufgebaute Bürstenkopf 5 linear oszillierend bewegt werden. Die Pumpe 18 erzeugt einen Strom der Spülflüssigkeit, der über die Ausspritzöffnungen 9 in Richtung der elastisch verformbaren Borsten 6 ausgestoßen wird. Mit Hilfe der Blende kann ein pulsierender Strom der Spülflüssigkeit erzeugt werden. Mit der chirurgischen Reinigungsvorrichtung kann so eine mechanische Reinigung bei gleichzeitiger Einwirkung des Flüssigkeitsstrahls der Spülflüssigkeit erfolgen.

Die gesamte chirurgische Reinigungsvorrichtung kann aus Kunststoff gefertigt werden und kann nach Gebrauch rückstandsfrei verbrannt werden. Es schadet allerdings auch nicht sehr viel, wenn einzelne kleinere Teile, wie beispielsweise das Rückstellelement 32 aus einem anderen Material, wie beispielsweise Stahl, gefertigt sind.

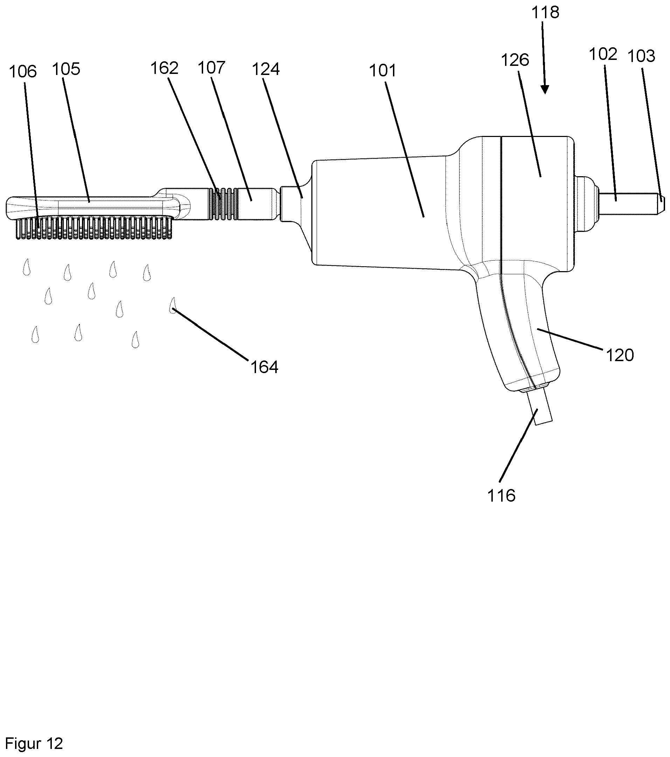

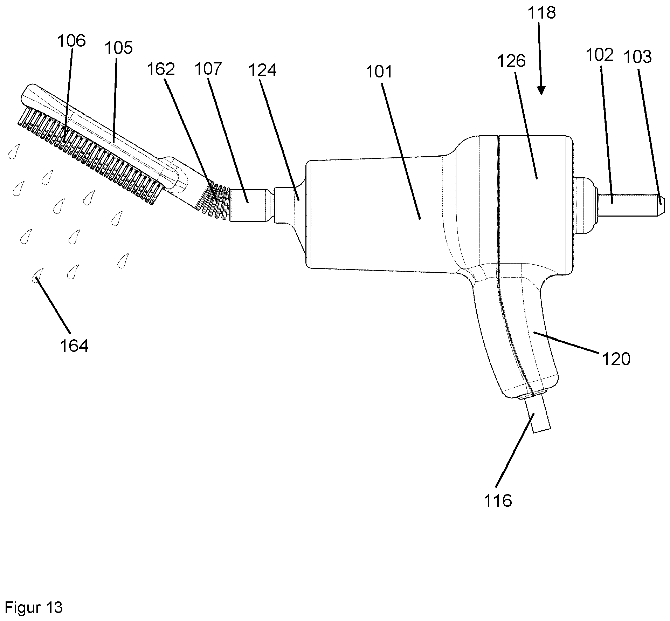

Die Figuren 12 und 13 zeigen eine schematische Seitenansicht einer beispielhaften zweiten erfindungsgemäßen chirurgischen Reinigungsvorrichtung. Die chirurgische Reinigungsvorrichtung kann ein Gehäuse 101 aufweisen, das die chirurgische Reinigungsvorrichtung weitgehend nach außen abschließt und in einem hinteren Bereich als Griffstück 120 zum Halten der chirurgischen Reinigungsvorrichtung ausgeformt werden kann. Der innere Aufbau der zweiten chirurgischen Reinigungsvorrichtung nach den Figuren 12 und 13 gleicht dem des ersten Ausführungsbeispiels.

Im Inneren des Gehäuses 101 befindet sich eine darin drehbar gelagerte Antriebswelle 102. Die Antriebswelle 102 weist ein hinteres Ende 103 auf, wobei das hintere Ende 103 direkt oder über eine Antriebswellenverlängerung 200 (siehe Figur 18) an eine externe Antriebseinheit (nicht gezeigt) angeschlossen oder anschließbar ist. Die externe Antriebseinheit kann beispielsweise ein im OP-Bereich üblicher Mehrzweck-Antrieb sein oder eine Bohrmaschine oder ähnliches.

An der Vorderseite der zweiten chirurgischen Reinigungsvorrichtung ist ein Bürstenkopf 105 angeordnet, wobei an dem Bürstenkopf 105 eine Vielzahl elastisch verformbarer Borsten 106 befestigt sein können.

Der Bürstenkopf 105 ist an einem vorderen Ende eines Rohrs 107 befestigt. Das Rohr 107 kann auch zur Einleitung von Spülflüssigkeit 164 in den Bürstenkopf 105 dienen. Der Bürstenkopf 105 kann als Hohlkörper geformt sein. Auf einer Unterseite des Bürstenkopfs 105 können mehrere Ausspritzöffnungen vorgesehen sein, die flüssigkeitsleitend mit dem Rohr 107 verbunden sein können. Hierzu können die Ausspritzöffnungen die Umgebung des Bürstenkopfs 105 mit einem Innenraum des Bürstenkopfs 105 verbinden, wobei der Innenraum des Hohlkörpers beziehungsweise des Bürstenkopfs 105 mit dem Rohr 107 flüssigkeitsleitend verbunden ist. Bevorzugt mündet das Rohr 107 in den Innenraum des Hohlkörpers beziehungsweise des Bürstenkopfs 105. Die Ausspritzöffnungen münden im Bereich der elastische verformbaren Borsten 106 in deren Wirkbereich, das heißt in den Bereich, den die elastisch verformbaren Borsten 106 bei einer linear oszillierenden Bewegung des Bürstenkopfs 105 überstreichen.

Im Unterschied zum ersten Ausführungsbeispiel weist das Rohr 107 eine flexibel knickbaren Rohrabschnitt 162 auf, mit dem der Winkel des Bürstenkopfs 105 gegen das Gehäuse 101 um bis zu 45° geneigt werden kann. Hierdurch können ansonsten schwer zugängliche Bereiche mit dem Bürstenkopf 105 erreicht werden.

Zwischen dem Rohr 107 und der Antriebswelle 102 ist ein Getriebe (nicht zu sehen) innerhalb des Gehäuses 101 angeordnet, das eine Drehung der Antriebswelle 102 in eine lineare oszillierende Bewegung des Rohrs 107 mit dem Bürstenkopf 105 übersetzt. Hierzu kann das Getriebe auf ein hinteres Ende des Rohrs 107 einwirken. Die Spülflüssigkeit 164 kann anschließend durch das Rohr 107 und durch den Bürstenkopf 105 zu den Ausspritzöffnungen fließen und von dort in den Wirkbereich der Borsten 106 gespritzt werden. Das Rohr 107 kann über eine erste Flüssigkeitsleitung (nicht zu sehen) in Form eines Schlauchs mit einer Pumpe 118 verbunden sein. Der Schlauch kann die lineare oszillierende Bewegung des Rohrs 107 gegen das Gehäuse 101 durch eine Verformung des Schlauchs bei der Bewegung des Rohrs 107 ausgleichen.

Die Antriebswelle 102 kann am hinteren Ende 103 als Anschluss in Form eines Sechskants oder einer anderen Mehrkanntstange ausgebildet sein. An das hintere Ende 103 beziehungsweise den Anschluss kann ein Spannfutter oder ein passender Gegenanschluss der externen Antriebseinrichtung angeschlossen sein oder werden. Eine Antriebswellenverlängerung 200 (siehe Figur 18) kann zwischen das hintere Ende 103 beziehungsweise den Anschluss und den Gegenanschluss der externen Antriebseinrichtung angeschlossen werden.

Die chirurgische Reinigungsvorrichtung kann eine Pumpe 118 aufweisen, die mit der Antriebswelle 102 im Bereich des hinteren Endes 103 der Antriebswelle 102 verbunden sein kann. Die Pumpe 118 kann über die Antriebswelle 102 angetrieben werden und kann zu diesem Zweck mit der Antriebswelle 102 verbunden sein. Die Pumpe 118 kann bevorzugt als Impellerpumpe ausgeführt sein (analog Figur 6). Die Pumpe 118 ist an eine zweite Flüssigkeitsleitung 116 zum Zuführen einer Flüssigkeit in die Pumpe 118 und an die erste Flüssigkeitsleitung zum Ableiten der Spülflüssigkeit 164 aus der Pumpe 118 in das Rohr 107 angeschlossen. Anstatt der Impellerpumpe kann auch eine andere Pumpe verwendet werden. Die Impellerpumpe ist jedoch aufgrund ihres einfachen Aufbaus und aufgrund der Tatsache, dass die Impellerpumpe bei einem Stillstand der Impellerpumpe die zweite Flüssigkeitsleitung 116 von der ersten Flüssigkeitsleitung physikalisch trennt, besonders bevorzugt.

Das Gehäuse 101 kann an seiner Unterseite als Griffstück 120 geformt sein. Das Griffstück 120 kann zur einfachen Handhabung der chirurgischen Reinigungsvorrichtung nach Art eines Pistolengriffs ausgeführt sein. Das Gehäuse 101 kann an seiner Vorderseite mit einem vorderen Verschluss 124 verschlossen sein. Dies erleichtert den Zusammenbau der chirurgischen Reinigungsvorrichtung. Der vordere Verschluss 124 kann zur linear beweglichen Lagerung des Rohrs 107 gegen das Gehäuse 101 genutzt werden.

Die Pumpe 118 kann ein Pumpengehäuse 126 aus Kunststoff aufweisen. Das Pumpengehäuse 126 kann außen einen Teil des Gehäuses 101 bilden. Die Antriebswelle 102 kann über Dichtungen abgedichtet durch das Pumpengehäuse 126 geführt und von dem Pumpengehäuse 126 gelagert sein.

Am Pumpengehäuse 126 kann ein Anschluss zum Verbinden der zweiten Flüssigkeitsleitung 116 mit einer Zuleitung von einer Flüssigkeitsquelle (nicht gezeigt) ausgebildet sein. Das Pumpengehäuse 126 kann auf seiner Vorderseite mit einer Frontplatte gegen den restlichen Innenraum des Gehäuses 101 getrennt sein. Die Antriebswelle 102 kann über eine Lagerung in die Pumpe 118 geführt sein.

Durch die Drehung der Antriebswelle 102 kann dann die Pumpe 118 betrieben werden. Auf diese Weise erfolgt gleichzeitig mit der linearen Oszillation der elastisch verformbaren Borsten 106 das Pumpen einer Spülflüssigkeit 164 mit der Pumpe 118 und damit das Ausstoßen eines Flüssigkeitsstrahls der Spülflüssigkeit 164 in den Wirkbereich der elastisch verformbaren Borsten 106.

Mit dem Getriebe (nicht zu sehen) im Gehäuse 101 wird die Drehung der Antriebswelle 102 in eine oszillierende lineare Bewegung des Rohrs 107 und des Bürstenkopfs 105 übersetzt. Gleichzeitig kann die Drehung der Antriebswelle 102 zum Antreiben der Pumpe 118 verwendet werden. Die Antriebswelle 102 ist dazu drehbar in dem Gehäuse 101 gelagert und das Rohr 107 ist nicht drehbar und linear (axial bezogen auf das Rohr 107) beweglich gegen das Gehäuse 101 gelagert.

Die Pumpe 118 kann über die zweite Flüssigkeitsleitung 116 mit einem Spülflüssigkeitsreservoir (nicht gezeigt) der Spülflüssigkeit 164 verbunden werden. Die zweite chirurgische Reinigungsvorrichtung kann betrieben werden, indem sie mit dem hinteren Ende 103 der Antriebswelle 102 direkt oder über eine Antriebswellenverlängerung 200 mit der externen Antriebseinheit verbunden wird. Die externe Antriebseinheit treibt die Antriebswelle 102 an. Durch die Drehung der Antriebswelle 102 kann die Pumpe 118 betrieben werden und gleichzeitig der mit den elastisch verformbaren Borsten 106 aufgebaute Bürstenkopf 105 linear oszillierend bewegt werden. Die Pumpe 118 erzeugt einen Strom der Spülflüssigkeit 164, der über die Ausspritzöffnungen in Richtung der elastisch verformbaren Borsten 106 ausgestoßen wird. Mit der chirurgischen Reinigungsvorrichtung kann so eine mechanische Reinigung bei gleichzeitiger Einwirkung des Flüssigkeitsstrahls der Spülflüssigkeit 164 erfolgen.

Die gesamte zweite chirurgische Reinigungsvorrichtung kann aus Kunststoff gefertigt werden und kann nach Gebrauch rückstandsfrei verbrannt werden. Es schadet allerdings auch nicht, wenn einzelne kleinere Teile aus einem anderen Material gefertigt sind.

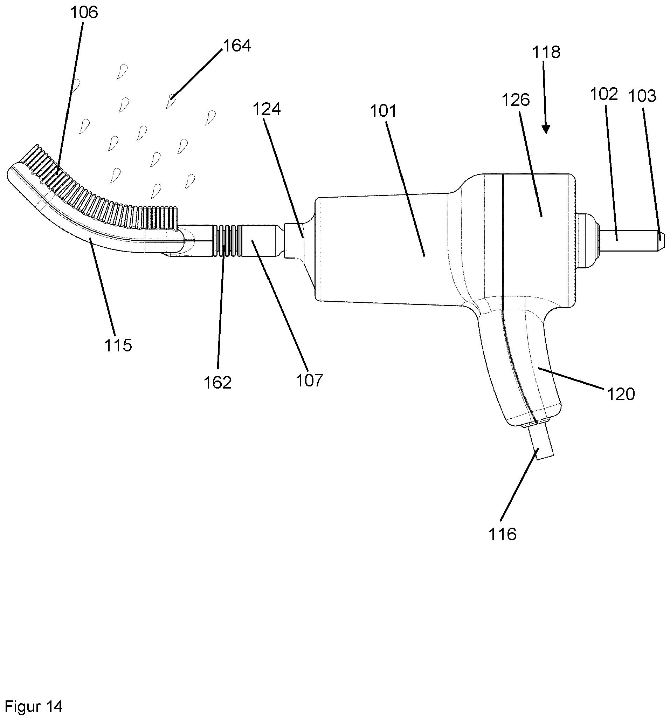

Figur 14 zeigt eine schematische perspektivische seitliche Ansicht einer dritten erfindungsgemäßen chirurgischen Reinigungsvorrichtung mit gekrümmtem Bürstenkopf 115. Bis auf den gekrümmten Bürstenkopf 115, der eine bessere Anwendung der chirurgischen Reinigungsvorrichtung an schwer zugänglichen Stellen ermöglicht, gleicht der Aufbau der dritten erfindungsgemäßen chirurgischen Reinigungsvorrichtung dem der zweiten erfindungsgemäßen chirurgischen Reinigungsvorrichtung nach den Figuren 12 und 13 sowie im Innerem dem Aufbaue der ersten erfindungsgemäßen chirurgischen Reinigungsvorrichtung nach den Figuren 1 bis 11.

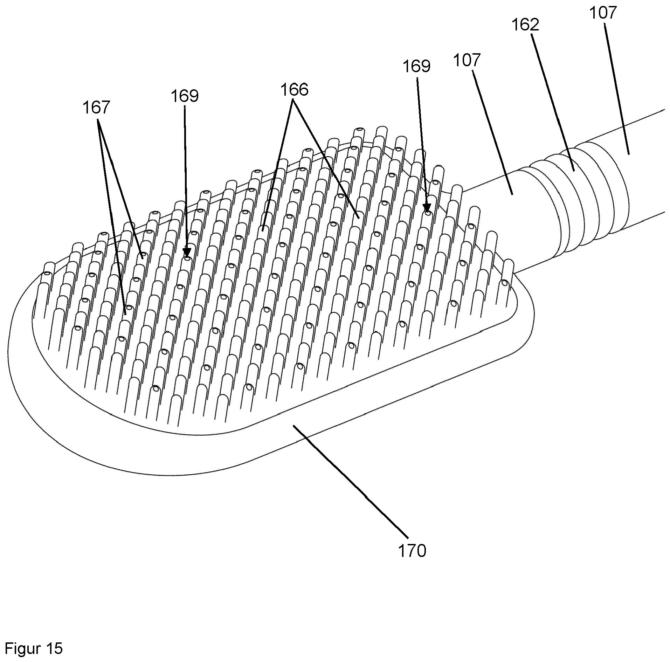

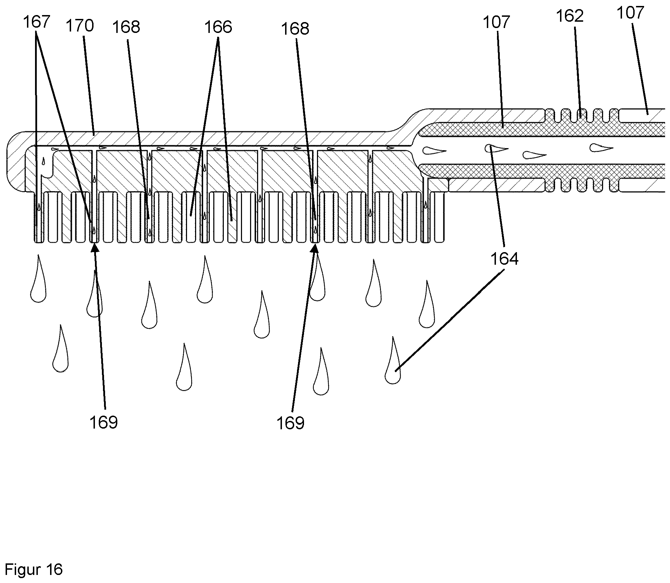

Figur 15 zeigt eine schematische perspektivische Ansicht eines Bürstenkopfs 170 für eine vierte erfindungsgemäße chirurgische Reinigungsvorrichtung mit massiven elastisch verformbaren Borsten 166 und mit hohlen elastisch verformbaren Borsten 167. Figur 16 zeigt hierzu eine schematische Querschnittansicht des Bürstenkopfs 170 für die vierte erfindungsgemäße chirurgische Reinigungsvorrichtung. Die hohlen elastisch verformbaren Borsten 167 sind an den Hohlraum des Bürstenkopfs 170 und damit an ein Rohr 107 angeschlossen. Im Inneren der hohlen elastisch verformbaren Borsten 167 sind Leitungen 168 vorgesehen, mit der die Spülflüssigkeit 164 bis zu Ausspritzöffnungen 169 an den von dem Bürstenkopf 170 abgewandten Enden der hohlen elastisch verformbaren Borsten 167 geleitet werden kann. Hierdurch kann der Ort der mechanischen Reinigung durch die elastisch verformbaren Borsten 166, 167 und der Ort des Einwirkens der Spülflüssigkeit 164 noch besser zusammengelegt werden und dadurch die Reinigungswirkung verbessert werden.

Ansonsten gleicht der Aufbau der vierten erfindungsgemäßen chirurgischen Reinigungsvorrichtung dem der zweiten erfindungsgemäßen chirurgischen Reinigungsvorrichtung nach den Figuren 12 und 13 sowie im Innerem dem Aufbaue der ersten erfindungsgemäßen chirurgischen Reinigungsvorrichtung nach den Figuren 1 bis 11.

Figur 17 zeigt eine schematische perspektivische Ansicht einer fünften erfindungsgemäßen chirurgischen Reinigungsvorrichtung mit einer Absaugeinrichtung. Die fünfte chirurgische Reinigungsvorrichtung nach Figur 17 ist genauso aufgebaut wie die zweite chirurgische Reinigungsvorrichtung nach den Figuren 12 und 13, wobei zusätzlich an dem Rohr 107 eine Absaugleitung 172 mit einer Halterung 174 befestigt ist. Die Absaugleitung 172 endet an einer Absaugöffnung 176, die im Bereich des Bürstenkopfs 105 angeordnet ist. Bevorzugt ist die Absaugöffnung 176 trichterförmig geformt. Gebrauchte Spülflüssigkeit 164 kann so aus dem Bereich des Bürstenkopfs 105 abgesaugt werden. Die Absaugleitung 172 kann zu diesem Zweck an eine externe Absaugpumpe angeschlossen sein.

Die Absaugleitung 172 kann mit einer Unterdruckquelle (nicht gezeigt) verbunden oder verbindbar sein und dient dem Entfernen der gebrauchten Spülflüssigkeit 164. Derartige Unterdruckquellen sind in Krankenhäusern üblicherweise vorhanden. Zwischen der Unterdruckquelle und der Absaugleitung 172 kann ein Abscheider (nicht gezeigt) zum Abtrennen fester Bestandteile aus der abgesaugten gebrauchten Spülflüssigkeit 164 angeordnet sein.

Die Absaugeinrichtung nach Figur 17 kann auch mit der ersten oder der dritten oder der vierten beispielhaften chirurgischen Reinigungsvorrichtung verwendet werden.

Figur 18 zeigt eine schematische perspektivische Außenansicht (Oben), eine schematische perspektivische Teilschnittansicht (Mitte) und eine schematische Querschnittansicht (Unten) einer Antriebswellenverlängerung 200, die Teil einer erfindungsgemäßen chirurgische Reinigungsvorrichtung sein kann oder über die eine erfindungsgemäße chirurgische Reinigungsvorrichtung angetrieben werden kann, indem die Antriebswellenverlängerung 200 das hintere Ende 3, 103 der Antriebswelle 2, 102 mit einer externen Antriebseinrichtung verbindet.

Die Antriebswellenverlängerung 200 kann einen beweglichen Schlauch 202 aufweisen, in dem eine bewegliche Welle 204, wie beispielsweise ein Seil, angeordnet sein kann. Der bewegliche Schlauch 202 und die bewegliche Welle 204 darin können vorzugsweise nach Art eines Schlauchs gebogen und/oder gekrümmt werden. Zur Verbindung mit dem hinteren Ende 3, 103 der Antriebswelle 2, 102 kann ein passendes Kantenloch 206 vorgesehen sein, in das das hintere Ende 3, 103 der Antriebswelle 2, 102 formschlüssig einsteckbar ist. Es kann auch eine magnetische Kupplung vorgesehen sein. Das Kantenloch 206 kann Teil einer Anschlussbuchse 208 sein, die starr mit einem Ende der beweglichen Welle 204 im Schlauch 202 verbunden ist. Am anderen Ende der beweglichen Welle 204 kann ein Verlängerungsanschluss 210 starr mit der beweglichen Welle 204 verbunden sein. Der Verlängerungsanschluss 210 kann mit einer externen Antriebseinheit verbunden sein oder verbunden werden und kann hierzu als Sechskant ausgeführt sein.

Die chirurgischen Reinigungsvorrichtungen können mit und ohne Absaugeinrichtung vollständig aus Kunststoff gefertigt sein. Hierdurch können die erfindungsgemäßen chirurgischen Reinigungsvorrichtungen nach Gebrauch zusammen mit anderem Abfall aus dem OP-Bereich entsorgt und auf hygienische vollständig Weise verbrannt werden. Gleichzeitig können die erfindungsgemäßen chirurgischen Reinigungsvorrichtungen vor dem Gebrauch auf einfache Weise mit Hilfe von Ethylenoxid oder mit Gamma-Strahlen desinfiziert werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 101: Gehäuse
- 2, 102: Antriebswelle
- 3, 103: Hinteres Ende der Antriebswelle
- 4: Vorderes Ende der Antriebswelle
- 5, 105, 115: Bürstenkopf
- 6, 106, 166: Elastisch verformbare Borsten
- 7, 107: Rohr
- 8: vorderes Ende des Rohrs
- 9, 169: Ausspritzöffnung
- 10: Getriebe
- 12: hinteres Ende des Rohrs
- 14: erste Flüssigkeitsleitung
- 16, 116: zweite Flüssigkeitsleitung
- 18, 118: Pumpe
- 20, 120: Griffstück
- 22: Stutzen
- 24, 124: Vorderer Verschluss
- 26, 126: Pumpengehäuse
- 28: Erste Scheibe
- 30: Zweite Scheibe
- 32: Rückstellelement
- 34: Impellerflügel
- 36: Lager
- 38: Flügel
- 40: Verschlusskappe
- 42: Gewinde
- 44: Frontplatte
- 46: Hinterer Schaft
- 48: Vorderer Schaft
- 50: Einmündung in das Rohr
- 52: Anschluss für die erste Flüssigkeitsleitung an die Pumpe
- 54: Einmündung in die erste Flüssigkeitsleitung
- 56: Einmündung in die Pumpe
- 58: Aufnahme
- 60: Wandung
- 162: flexibel knickbarer Rohrabschnitt
- 164: Spülflüssigkeit
- 167: Hohle elastisch verformbare Borste
- 168: Leitung
- 170: Bürstenkopf
- 172: Absaugleitung
- 174: Halterung
- 176: Absaugöffnung
- 200: Antriebswellenverlängerung
- 202: Schlauch
- 204: Bewegliche Welle
- 206: Kantenloch
- 208: Anschlussbuchse
- 210: Verlängerungsanschluss

## Patentansprüche

1. Chirurgische Reinigungsvorrichtung aufweisend
ein Gehäuse (1, 101),
eine Antriebswelle (2, 102), wobei die Antriebswelle (2, 102) drehbar in dem Gehäuse (1, 101) gelagert ist, wobei die Antriebswelle (2, 102) ein hinteres Ende (3, 103) zur Verbindung mit einer Antriebseinheit aufweist und wobei die Antriebswelle (2, 102) ein vorderes Ende (4) aufweist, das dem hinteren Ende (3, 103) gegenüberliegend angeordnet ist,
einen Bürstenkopf (5, 105, 115, 170), wobei der Bürstenkopf (5, 105, 115, 170) eine Vielzahl elastisch verformbarer Borsten (6, 106, 166, 167) aufweist, die an dem Bürstenkopf (5, 105, 115, 170) befestigt sind, und wobei der Bürstenkopf (5, 105, 115, 170) außerhalb des Gehäuses (1, 101) angeordnet ist,
eine Pumpe (18, 118) zum Pumpen einer Spülflüssigkeit (164), wobei die Pumpe (18, 118) mit der Antriebswelle (2, 102) verbunden ist und von der Antriebswelle (2, 102) anzutreiben ist,
ein Rohr (7, 107), wobei der Bürstenkopf (5, 105, 115, 170) an einem vorderen Ende (8) des Rohrs (7, 107) angeordnet ist, wobei das Rohr (7, 107) linear beweglich gegen das Gehäuse (1, 101) gelagert ist und wobei der Bürstenkopf (5, 105, 115, 170) im Bereich der elastisch verformbaren Borsten (6, 106, 166, 167) zumindest eine Ausspritzöffnung (9, 169) aufweist, wobei das Rohr (7, 107) flüssigkeitsdurchlässig durch die zumindest eine Ausspritzöffnung (9, 169) in Richtung der elastisch verformbaren Borsten (6, 106, 166, 167) mündet,
ein Getriebe (10), wobei das Getriebe (10) mit dem vorderen Ende (4) der Antriebswelle (2, 102) und mit einem hinteren Ende (12) des Rohrs (7, 107) verbunden ist und wobei das Getriebe (10) die Drehbewegung der Antriebswelle (2, 102) in eine oszillierende lineare Bewegung des Rohrs (7, 107) und des Bürstenkopfs (5, 105, 115, 170) übersetzt.

2. Chirurgische Reinigungsvorrichtung nach Anspruch 1, **dadurch gekennzeichnet, dass** die chirurgische Reinigungsvorrichtung eine erste Flüssigkeitsleitung (14) aufweist, wobei die erste Flüssigkeitsleitung (14) mit dem hinteren Ende (12) des Rohrs (7, 107) flüssigkeitsdurchlässig verbunden ist und am hinteren Ende (12) des Rohrs (7, 107) fixiert ist und an einem dem hinteren Ende (12) des Rohrs (7, 107) gegenüberliegenden Ende relativ zum Gehäuse (1, 101) fixiert ist,
wobei bevorzugt die erste Flüssigkeitsleitung (14) ein flexibler beweglicher Schlauch ist, der an einem ersten Ende des Schlauchs fest mit dem hinteren Ende (12) des Rohrs (7, 107) verbunden ist.

3. Chirurgische Reinigungsvorrichtung nach einem der vorangehenden Ansprüche, **dadurch gekennzeichnet, dass**
die chirurgische Reinigungsvorrichtung eine zweite Flüssigkeitsleitung (16, 116) aufweist, wobei die zweite Flüssigkeitsleitung (16, 116) derart mit der Pumpe (18, 118) verbunden ist, dass eine Spülflüssigkeit (164) von der Pumpe (18, 118) durch die zweite Flüssigkeitsleitung (16, 116) in die Pumpe (18, 118) ansaugbar ist und wobei die erste Flüssigkeitsleitung (14) derart mit der Pumpe (18, 118) verbunden ist, dass eine Spülflüssigkeit (164) von der Pumpe (18, 118) aus der Pumpe (18, 118) heraus in die zweite Flüssigkeitsleitung (16, 116) drückbar ist, wobei vorzugsweise die Pumpe (18, 118) derart mit der Antriebswelle (2, 102) verbunden ist, dass zumindest bei einem Stillstand der Antriebswelle (2, 102) die zweite Flüssigkeitsleitung (16, 116) in der Pumpe (18, 118) von der erste Flüssigkeitsleitung (14) flüssigkeitsundurchlässig getrennt ist oder abgeriegelt ist, wobei bevorzugt bei einer Drehbewegung der Antriebswelle (2, 102) die zweite Flüssigkeitsleitung (16, 116) flüssigkeitsleitend mit der erste Flüssigkeitsleitung (14) verbunden ist.

4. Chirurgische Reinigungsvorrichtung nach einem der vorangehenden Ansprüche, **dadurch gekennzeichnet, dass**
die Pumpe (18, 118) eine selbstansaugende Pumpe (18, 118) ist, die unmittelbar mit der Antriebswelle (2, 102) verbunden ist, wobei die selbstansaugende Pumpe (18, 118) in dem Gehäuse (1, 101) angeordnet ist, und/oder
die Pumpe (18, 118) eine Impellerpumpe ist, die auf der Antriebswelle (2, 102) angeordnet ist, wobei die Impellerpumpe einen Rotor mit Impellerflügeln (34) aufweist, wobei der Rotor mit den Impellerflügeln (34) kraft- und/oder formschlüssig mit der Antriebswelle (2, 102) verbunden ist.

5. Chirurgische Reinigungsvorrichtung nach einem der vorangehenden Ansprüche, **dadurch gekennzeichnet, dass**
das Gehäuse (1, 101) ein Griffstück (20) zum Halten der chirurgischen Reinigungsvorrichtung mit einer Hand aufweist und wobei das Gehäuse (1, 101) eine Öffnung für das hintere Ende (3, 103) der Antriebswelle (2, 102) aufweist, und/oder die Antriebswelle (2, 102) durch eine separate Antriebseinheit drehbar und anzutreiben ist, wobei die separate Antriebseinheit hierzu mit dem hinteren Ende (3, 103) der Antriebswelle (2, 102) verbunden oder verbindbar ist, insbesondere lösbar verbunden oder verbindbar ist.

6. Chirurgische Reinigungsvorrichtung nach einem der vorangehenden Ansprüche, **dadurch gekennzeichnet, dass**
die chirurgische Reinigungsvorrichtung aus Kunststoff gefertigt ist, wobei bevorzugt die gesamte chirurgische Reinigungsvorrichtung durch Verbrennung rückstandsfrei entsorgbar ist, und/oder
die chirurgische Reinigungsvorrichtung eine Absaugeinrichtung mit zumindest einer Absaugöffnung (176) im Bereich der elastisch verformbaren Borsten (6, 106, 166, 167) aufweist, wobei die Absaugeinrichtung eine Absaugleitung (172) aufweist, die an eine Unterdruckquelle anschließbar ist oder angeschlossen ist, wobei bevorzugt die Absaugeinrichtung an einen Abscheider zum Abscheiden von festen Bestandteilen aus der gebrauchten Spülflüssigkeit (164) angeschlossen oder anschließbar ist und/oder die Unterdruckquelle eine Vakuumpumpe aufweist, die ein Teil der chirurgischen Reinigungsvorrichtung ist und die von der Antriebswelle (2, 102) antreibbar ist

7. Chirurgische Reinigungsvorrichtung nach einem der vorangehenden Ansprüche, **dadurch gekennzeichnet, dass**
die chirurgische Reinigungsvorrichtung eine flexible Antriebswellenverlängerung (200) aufweist, wobei die flexible Antriebswellenverlängerung (200) an das hintere Ende (3, 103) der Antriebswelle (2, 102) angeschlossen oder anschließbar ist und wobei die flexible Antriebswellenverlängerung (200) an eine externe Antriebseinheit anschließbar ist, so dass die Antriebswelle (2, 102) mit der externen Antriebseinheit über die flexible Antriebswellenverlängerung (200) gegen das Gehäuse (1, 101) drehbar ist, und/oder der Bürstenkopf (5, 105, 115, 170) ein Hohlkörper ist und die zumindest eine Ausspritzöffnung (9, 169) einen Innenraum des Hohlkörpers mit der Umgebung im Bereich der elastisch verformbaren Borsten (6, 106, 166, 167) verbindet, wobei das Rohr (7, 107) über den Innenraum des Hohlkörpers in die zumindest eine Ausspritzöffnung (9, 169) mündet.

8. Chirurgische Reinigungsvorrichtung nach einem der vorangehenden Ansprüche, **dadurch gekennzeichnet, dass**
die chirurgische Reinigungsvorrichtung ein Rückstellelement (32) aufweist, mit dem das Rohr (7, 107) in Richtung der Antriebswelle (2, 102) gedrückt wird, wobei bevorzugt das Rückstellelement (32) zumindest eine Spiralfeder ist, wobei besonders bevorzugt die zumindest eine Spiralfeder um das Rohr (7, 107) herum und/oder um einen Schaft (48) zur Verbindung des Rohrs (7, 107) mit dem Getriebe (10) herum angeordnet ist und gegen das Gehäuse (1, 101) gelagert ist, wobei vorzugsweise das Getriebe (10) eine erste Scheibe (28) aufweist, die mit dem vorderen Ende (4) der Antriebswelle (2, 102) verbunden ist oder die an dem vorderen Ende (4) der Antriebswelle (2, 102) befestigt ist, wobei die erste Scheibe (28) schräg zur Drehachse der Antriebswelle (2, 102) gestellt ist, und das Getriebe (10) eine zweite Scheibe (30) aufweist, wobei die zweite Schreibe (30) mit dem hinteren Ende (12) des Rohrs (7, 107) verbunden ist oder die zweite Schreibe (30) an dem hinteren Ende (12) des Rohrs (7, 107) befestigt ist, und wobei die zweite Scheibe (30) schräg zur Längsachse des Rohrs (7, 107) oder schräg zur Drehachse der Antriebswelle (2, 102) gestellt ist und wobei die zweite Scheibe (30) durch das elastische Rückstellelement (32) gegen die erste Scheibe (28) gedrückt wird.

9. Chirurgische Reinigungsvorrichtung nach einem der vorangehenden Ansprüche, **dadurch gekennzeichnet, dass**
das Getriebe (10) eine Scheibe an dem vorderen Ende (4) der Antriebswelle (2, 102) oder an dem hinteren Ende (12) des Rohrs (7, 107) aufweist, wobei die Scheibe eine gegen die Drehachse der Antriebswelle (2, 102) geneigte Oberfläche aufweist, oder das Getriebe (10) eine erste Scheibe (28) an dem vorderen Ende (4) der Antriebswelle (2, 102) und eine zweite Scheibe (30) an dem hinteren Ende (12) des Rohrs (7, 107) aufweist, wobei die erste Scheibe (28) und die zweite Scheibe (30) jeweils eine gegen die Drehachse der Antriebswelle (2, 102) geneigte Oberfläche aufweisen, wobei die geneigten Oberflächen der ersten Scheibe (28) und der zweiten Scheibe (30) zueinander ausgerichtet sind.

10. Chirurgische Reinigungsvorrichtung nach einem der vorangehenden Ansprüche, **dadurch gekennzeichnet, dass**
der Bürstenkopf (5, 105, 115, 170) über eine lösbare Verbindung mit dem Rohr (7, 107) verbunden ist und/oder
zwischen dem Bürstenkopf (5, 105, 115, 170) und dem Rohr (7, 107) oder in dem Rohr (7, 107) ein flexibel knickbarer Rohrabschnitt (162) angeordnet ist, der ein Abknicken des Bürstenkopfs (5, 105, 115, 170) gegen die Längsachse des Rohrs (7, 107) um bis zu 45° zulässt, wobei bevorzugt der flexibel knickbare Rohrabschnitt (162) als Muffe ausgebildet ist.

11. Chirurgische Reinigungsvorrichtung nach einem der vorangehenden Ansprüche, **dadurch gekennzeichnet, dass**
eine Führung im Gehäuse (1, 101) eine Drehbewegung des Rohrs (7, 107) gegen das Gehäuse (1, 101) verhindert, wobei vorzugsweise das Gehäuse (1, 101) oder eine Führung des Gehäuses (1, 101) die lineare Bewegung des Rohrs (7, 107) führt, und/oder
zumindest eine rohrförmige elastische Borste (167) als eine der zumindest einen elastischen Borsten (6, 106, 166, 167) vorgesehen ist, wobei die zumindest eine rohrförmige elastische Borste (167) innen hohl ist und mit dem Rohr (7, 107) flüssigkeitsdurchlässig verbunden ist, wobei vorzugsweise eine freie Öffnung der zumindest einen rohrförmigen elastischen Borste (167) eine Ausspritzöffnung (169) der zumindest einen Ausspritzöffnung (9, 169) ist.

12. Chirurgische Reinigungsvorrichtung nach einem der vorangehenden Ansprüche, **dadurch gekennzeichnet, dass**
zumindest eine Nocke auf der Antriebswelle (2, 102) befestigt ist und ein elastisch verformbarer Schlauch an das hintere Ende (12) des Rohrs (7, 107) angeschlossen ist, wobei der elastisch verformbare Schlauch derart im Wirkbereich der Nocken angeordnet ist, dass bei einer vollständigen 360°-Drehung der Antriebswelle (2, 102) um ihre Längsachse ein Spülflüssigkeitsstrom durch den elastisch verformbaren Schlauch mindestens einmal pro Umdrehung der Antriebswelle (2, 102) unterbrochen wird und dadurch pulsierende Spülflüssigkeitsstrahlen aus der zumindest einen Ausspritzöffnung (9, 169) austreten, und/oder
die zumindest eine Ausspritzöffnung (9, 169) derart geformt und derart relativ zu den elastisch verformbaren Borsten (6, 106, 166, 167) angeordnet ist, dass ein durch die zumindest eine Ausspritzöffnung (9, 169) ausgestoßener Strahl der Spülflüssigkeit (164) in den Wirkbereich der linear oszillierenden elastisch verformbaren Borsten (6, 106, 166, 167) trifft.

13. Verfahren zum Betreiben einer chirurgischen Reinigungsvorrichtung, wobei das Verfahren die folgenden Schritte aufweist:
A) Drehen einer Antriebswelle (2, 102) der chirurgischen Reinigungsvorrichtung in einem Gehäuse (1, 101) der chirurgischen Reinigungsvorrichtung;
B) Übersetzen der Drehung der Antriebswelle (2, 102) in eine lineare oszillierende Bewegung eines Rohrs (7, 107) und einem an einem vorderen Ende (8) des Rohrs (7, 107) befestigten Bürstenkopfs (5, 105, 115, 170) mit einem Getriebe (10), wobei das Rohr (7, 107) linear beweglich in dem Gehäuse (1, 101) gelagert ist;
C) Pumpen einer Spülflüssigkeit (164) durch das linear oszillierende Rohr (7, 107) zu zumindest einer Ausspritzöffnung (9, 169) im Bürstenkopf (5, 105, 115, 170), wobei das Pumpen der Spülflüssigkeit (164) mit einer Pumpe (18, 118) durchgeführt wird, wobei die Pumpe (18, 118) mit der sich drehenden Antriebswelle (2, 102) angetrieben wird; und
D) Einspritzen der aus der zumindest einen Ausspritzöffnung (9, 169) in den Wirkbereich der elastisch verformbaren Borsten (6, 106, 166, 167).

14. Verfahren nach Anspruch 13, **dadurch gekennzeichnet, dass** das Verfahren mit einer chirurgischen Reinigungsvorrichtung nach einem der Ansprüche 1 bis 12 durchgeführt wird.

15. Verfahren nach Anspruch 13 oder 14, **dadurch gekennzeichnet, dass** die Spülflüssigkeit (164) ungepulst oder mit der sich drehenden Antriebswelle (2, 102) gepulst in den Wirkbereich der linear oszillierenden elastisch verformbaren Borsten (6, 106, 166, 167) gesprüht wird, und/oder
vor Schritt A) die chirurgische Reinigungsvorrichtung, insbesondere das Rohr (7, 107) oder die Pumpe (18, 118), mit einem Spülflüssigkeitsreservoir verbunden wird.

16. Verfahren nach einem der Ansprüche 13 bis 15, **dadurch gekennzeichnet, dass** die gebrauchte Spülflüssigkeit (164) aus dem Bereich der Borsten (6, 106, 166, 167) zumindest teilweise mit einer Absaugeinrichtung abgesaugt wird, wobei bevorzugt nach der Absaugung mit einem Abscheider feste Partikel der gebrauchten Spülflüssigkeit (164) von den flüssigen Bestandteilen getrennt werden, und/oder vor Schritt A) die chirurgische Reinigungsvorrichtung an eine Antriebseinheit angeschlossen wird, wobei die Drehung der Antriebswelle (2, 102) in den Schritten A) bis D) mit der Antriebseinheit angetrieben wird, wobei bevorzugt die Antriebseinheit an ein hinteres Ende (3, 103) der Antriebswelle (2, 102) angeschlossen wird.

17. Verfahren nach einem der Ansprüche 13 bis 16, **dadurch gekennzeichnet, dass** in Schritt B) eine gegen die Drehachse der Antriebswelle (2, 102) geneigte erste Scheibe (28), die an einem vorderen Ende (4) der Antriebswelle (2, 102) befestigt ist, auf dem hinteren Ende (12) des linear beweglichen Rohrs (7, 107) oder auf einer gegen die Drehachse der Antriebswelle (2, 102) geneigte zweite Scheibe (30) abrollt, wobei bevorzugt die zweite Scheibe (30) mit einem Rückstellelement (32) gegen die erste Scheibe (28) gedrückt wird.
